# NEW EUROPEAN PATENT SPECIFICATION

(11) **EP 0 839 139 B2**
(45) Date of publication and mention of the opposition decision: **18.11.2009**
(45) Mention of the grant of the patent: 03.09.2003
(21) Application number: 96923544.9
(22) Date of filing: 28.06.1996
(51) Int. Cl.: C07D 233/54, H01M 10/40, H01M 6/16

(54) **HYDROPHOBIC IONIC LIQUIDS**
HYDROPHOBE IONISCHE FLÜSSIGKEITEN
LIQUIDES IONIQUES HYDROPHOBES

(30) Priority: 30.06.1995 US 497310
(43) Date of publication of application: 06.05.1998
(73) Proprietor: COVALENT ASSOCIATES INCORPORATED, Woburn, MA 01801 (US)
(72) Inventor: KOCH, Victor, R., Lincoln, MA 01773 (US); NANJUNDIAH, Chenniah, San Diego, CA 92129 (US); CARLIN, Richard, T., Nashua, NH 03063 (US)
(74) Representative: Hatzmann, Martin
(86) International application number: PCT/US1996/011097
(87) International publication number: WO 1997/002252

(56) References cited:
- J. ELECTROCHEM. SOC., vol. 141, no. 7, 1994, pages L73-L76, XP000605410 R.T. CARLIN ET AL.: "Dual intercalating molten electrolyte batteries"
- CHEMICAL ABSTRACTS REGISTRY HANDBOOK - NUMBER SECTION., 1993, COLUMBUS US, pages 4899rv-4906rv, XP002016355 & CHEMICAL ABSTRACTS, vol. 120, no. 16, 1994 Columbus, Ohio, US; abstract no. 206757g, YUKIHIRO ET AL.: "Molten salt electrolytes for electrolytic capacitors" page 1135;
- J. ELECTROCHEM. SOC. , vol. 142, no. 7, July 1995, pages L116-L118, XP000605411 V.R. KOCH ET AL.: "The interfacial stability of Li with two new solvent-free ionic liquids"
- INORG. CHEM., vol. 35, January 1996, pages 1168-1178, XP000605807 P. BONHÔTE ET AL.: "Hydrophobic, highly conductive ambient-temperature molten salts"

## Description

### FIELD OF THE INVENTION

This invention relates to solvent-free ionic liquids, useful as non-aqueous electrolytes, particularly in non-aqueous batteries, electrochemical capacitors, electroplating, catalysis and chemical separations.

### BACKGROUND OF THE INVENTION

Solvent-free ionic liquids or "room temperature molten salts" were first disclosed by Hurley and Wier in a series of U.S. Patents (2,446,331; 2,446,349; 2,446,350). These room temperature melts, comprised of AlCl₃ and a variety of n-alkylpyridinium halides, afforded a conducting bath for aluminum electroplating. However, a disadvantage of these first molten salts, and a serious problem with any solvent-free ionic liquid containing a strong Lewis acid such as AlCl₃, is the liberation of toxic gas when exposed to moisture. Additionally, the highly reactive nature of Lewis acids used to form room temperature melts limits the kinds of organic and inorganic compounds which are stable in these media.

Over the past 15 years, work in room temperature melts has been dominated by the use of varying proportions of AlCl₃ and 1-ethyl-3-methylimidazolium (EMI) chloride as discussed in separate review articles by Wilkes and Osteryoung (Osteryoung, Robert A., (p.329) and Wilkes, John S., (p.217) in Molten Salt Chemistry, G. Mamantov and R. Marassi, eds., D. Reidel Publishing, Dordrecht, Holland, 1987) and in Japanese patent Nos. 0574656 (Endo, 1993) and 0661095 (Kakazu, 1994). However, these solvent-free ionic media suffer from the same problems encountered in the melts disclosed by Hurley and Wier.

In 1992, Wilkes and Zaworotko disclosed two new room temperature solvent-free ionic liquids, EMI BF₄ and EMI O₂CCH₃ (J. Chem. Soc., Chem. Commun., 965, 1992). Although neither liquid contains a Lewis acid, and therefore, does not liberate toxic gas when exposed to moisture, both are hygroscopic. Additionally, as electrolytes in electrochemical cells, the BF₄ and CH₃CO₂ anions oxidize at potentials lower than those desirable for electrochemical generators.

Similarly, Carlin et al., J. Electrochem. Soc., Vol. 141, No. 7, July 1994, pp. L73-L76, describes in connection with dual intercalating molten electrolyte batteries, molten salts using 1-ethyl-3-methylimidazolium (EMI⁺) or 1, 2-dimethyl-3-propylimidazolium (DMPI⁺) as the cation and ALCL₄, BF₄, CF₃SO₃, or C₆H₅CO₂ as the anion. However, salts with those anions contain Lewis acids and/or are soluble in or reactive with water, and therefore experience the attendant problems described in the preceding paragraphs.

J. Cas 120(16), 1994 abstract 2067579 mentions pyridinium and imidazolinium salts, paired with ethanedioate, butenedioate or 1,2-benzenedicarboxylate anions.

J. Electrochem. Soc., 142(7), 1995, L116-L118, July 1995 discloses solvent-free ionic liquids comprising imide or methide salts of 1,2-dimethyl-3-propylimidazolium.

In org. Chem., 35, 1996, 1168-1178, January 1996, discloses dialkylimidazolium salts with varius anions.

Therefore, a need exists to improve solvent-free ionic liquids such that they exhibit wide electrochemical windows and are not hygroscopic. Such improved solvent-free ionic liquids are the subject of the present invention.

EP-A 718288, published on June 26, 1996, describes imidazolium imides as hydrophobic ionic liquid and their use in electrochemical cels.

V. Beyl et al, Liebigs Ann. Chem. 731 58-66 mentions the synthesis of N-methylpyridiniumperfluorobutylsulfonate and N-methylimidazoleperfluorobutylsulfonate.

US. A 4,007,150 mentions the use of N-methylpyridiniumperfluorobutane- and octane sulfonate, as mould release agents.

WO-A 93/09092 relates to bis(perfluorosulfonyl) methanes (methide) and their use in electrochemical devices.

EP 699349, published as WO-A 95/26056 on September 28, 1995 covers solutions of compounds represented by the formula (1/mM)⁺[(ZY)₂N]⁻ , (1/mM)⁺[(ZY)₃C]⁻ , (1/mM)⁺[(ZY)₂CQ]⁻, wherein M represents a cation selected from pyridines and imidazoles, Y represents SO₂ or POZ, Q represents H, COZ or Z and each substituent Z represents independently a fluorine atom, or an organic moiety which may or may not be perfluorinated, which moiety optionally contains at least one polymerizable function, with the proviso that at least one of the Z-substituents represents a fluorine atom, in an aprotic solvent.

### SUMMARY OF THE INVENTION

The invention provides for a hydrophobic ionic liquid as defined in claim 1.

According to the present invention, hydrophobic ionic liquids are provided having improved properties for application in non-aqueous batteries, electrochemical capacitors, electroplating, catalysis and chemical separations. When fluorinated, the hydrophobic ionic liquids of the invention are also particularly useful as superior hydraulic fluids and inert liquid diluents for highly reactive chemicals.

The ionic liquids of the invention have a wide liquidus range and offer the advantages of high thermochemical and electrochemical stability. In addition, the ionic liquids of the invention are hdrophobic in nature, being poorly soluble in water. This finding is of great technological importance since the presence of water in, e.g. electrochemical generators can severly shorten the lifetime of the device. Similarly, the presence of water in certain electroplating baths results in a poor metal deposition. In addition, as taught by U.S. patent 5,220,106, the use of water with a solvent-free ionic liquid (e.g., for petroleum separations) is highly desirable, but the presence of water diminishes extraction capacity because the ionic liquid dissolves into the water cosolvent.

The hydrophobic, ionic liquids of the invention as defined in claim 1 may have one of the following formulas: 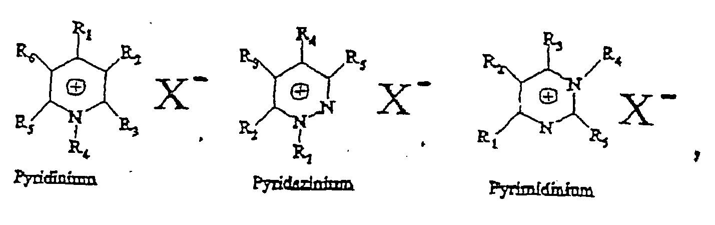 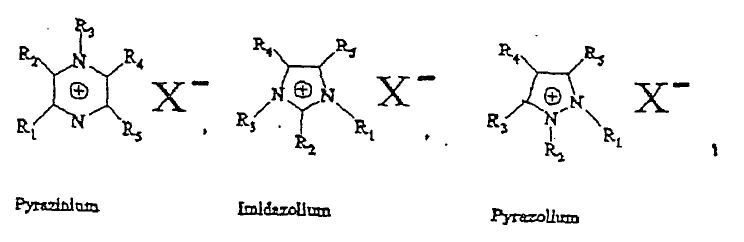 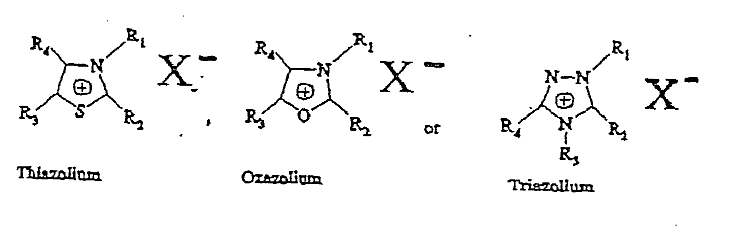 wherein R₁, R₂, R₃, R₄, R₅, and R₆ are either H; F; separate alkyl groups of from 1 to 4 carbon atoms, respectively, or joined together to constitute a unitary alkylene radical of from 2 to 4 carbon atoms forming a ring structure converging on N; or separate phenyl groups; and wherein the alkyl groups, alkylene radicals or phenyl groups may be substituted with electron withdrawing groups, preferably F-, Cl-, CF₃-, SF₅-, CF₃S-, (CF₃)₂CHS- or (CF₃)₃CS-; and X⁻ is a non-Lewis acid containing polyatomic anion having a van der Waals volume exceeding 100 Å³. Preferably, the van der Waals volume of the polyatomic anion X' exceeds 140 Å³.

An exemplary anion in the hydrophobic ionic liquids when used in combination with a lithium salt in an electrochemical cell of the invention is of the following form: 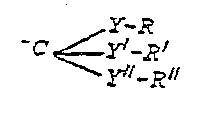 wherein each of the moieties Y, Y', and Y", are -SO₂- or -CO-; the groups R and R' are separate halogenated alkyl groups of 1-4 carbon atoms, respectively or are joined together to constitute a unitary halogenated alkylene radical of from 2-4 carbon atoms linking Y and Y' and forming a ring structure which includes R, R', Y, Y', and the carbon atom to which Y and Y' are attached; and the group R" is an alkyl or haloalkyl radical of 1-4 carbon atoms or a halogenated phenyl group.

In another exemplary anion of the hydrophobic ionic liquid of the invention, the -Y'-R' group in the formula above is replaced by Z, which is an electron-withdrawing substituent selected from the group consisting of -C(O)H, -NO₂, -CN, -F, and perfluorinated alkyls and aryls containing no more than 8 carbons.

A further exemplary anion has the formula: 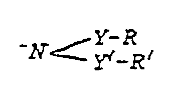 wherein each of the moieties Y and Y' are -SO₂- or -CO-; and the groups R and R' are separate halogenated alkyl groups of 1-4 carbon atoms.

In the exemplary anions described above, the moieties Y, Y', and Y" are preferably -SO₂- moieties. The groups R, R', and R" each preferably contains one or two carbon atoms, with one carbon atom being most preferred. These groups are preferably halogenated with fluorine or chlorine, the most preferred halogen being fluorine, and are preferably perhalogenated.

In other embodiments, the anion is a mono- or di-perfluorosulfonate, or the anion is any one of (CF₃)₂PF₄⁻,(CF₃)₃PF₃⁻, (CF₃)₄PF₂⁻,(CF₃)₅PF⁻,(CF₃)₆P⁻,SF₅CF₂SO₃⁻ ,SF₅CHFCF₂SO₃⁻, CF₃CF₂(CF₃)₂CO⁻, or [O(CF₃)₂C₂(CF₃)₂O]₂PO⁻.

Exemplary perfluorinated hydrophobic ionic liquids are: 1-ethyl-3-methylimidazolium perfluoro-1,1-dimethylpropyl alkoxide (EMI pfDMP, I) and perfluoro-1-ethyl-3-methylimidazolium imide (pfEMI Im, II).

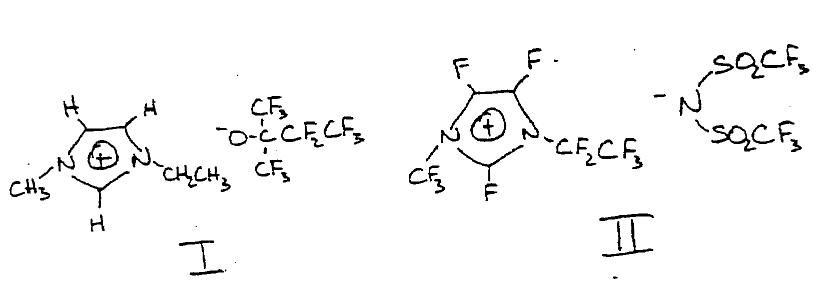

Not being bound by any theory, it is believed that one of the causes of the desirable hydrophobic property of the ionic liquids of the invention is the large size of the cations and anions involved. For example, the anion (CF₃SO₂)₂N⁻, bis-(trifluoromethylsulfonyl) imide or "Imide," has a van der Waals volume of 143, while the anion perfluoro-1,1-dimethylpropyl alkoxide has a van der Waals volume of 144 Å³. For comparison, other anions commonly paired with EMI cations in prior art ionic liquids are AlCl₄⁻ (113 Å³), CF₃SO₃⁻ (80 Å³), and BF₄⁻ (48 Å³), all of which are highly reactive with or highly soluble in water.

Exemplary imidazolium and pyridinium cations in the ionic liquids of the invention, 1-ethyl-3-methylimidazolium (EMI) and n-butylpyridinium (BP) have van der Waals volumes of 118 and 152 Å³, respectively. Additionally, the delocalization of the cation positive charge over the ring atoms results in a relatively low crystal lattice energy and a less reactive species. In the prior art, the imide and methide anions are commonly paired with metal cations (e.g., Li⁺, Na⁺ or K⁺) or the ammonium ion, which have van der Waals volumes in the range of about 2 to about 18 Å³. Properties of the imidazolium and pyridinium cations along with those of the other nitrogen-based cations described herein are discussed in Wilkes et al., U.S. Air Force Report No. FJSRL-TR-82-0002, U.S. Air Force Academy, January, 1982.

Furthermore, the hydrophobic ionic liquids of the invention are defined as containing only non-Lewis acid anions. This requirement reduces the reactive nature of the ionic liquids of the invention and is believed to contribute to the desirable hydrophobic property of the disclosed compounds.

When the cation and/or the anion is fluorinated, the hydrophobic ionic liquids of the invention have certain additional special properties including resistance to extremes of temperature and pressure, resistance to corrosive acids and bases, and inertness to organic solvents and oxidizing agents. For example, pfEMI Im can be likened to "ionic liquid Teflon®," in analogy with the physical and chemical properties of Teflon®, a solid perfluorinated hydrocarbon, (see, e.g., R.D. Chambers in "Fluorine in Organic Chemistry," Wiley-Interscience, New York, 1973, and references therein). Perfluorinated hydrophobic ionic liquids of the invention have applications as superior hydraulic fluids, inert liquid diluents for highly reactive chemicals and solvents with a high capacity for dissolved gases such as oxygen, and are useful for catalysis and for oil and gas separations where the desired product is partitioned between an aqueous and hydrophobic perfluorinated ionic liquid phase.

A further advantage of the hydrophobic ionic liquids of the invention is their ability to dissolve quantities of LiX (where X= any anion of the invention). The presence of the lithium cation then allows these media to be used as electrolytes in either primary or secondary (rechargeable) lithium batteries. For example, the dissolution of 250 mM, Lilm in 1,2-dimethyl-3-propylimidazolium (DMPI) Im results in a stable electrochemical interface in a metallic Li electrode dipped into this medium.

Another advantage of these new media is the unprecedented high oxidation potential afforded by the electrochemical stability of the anions. The Imide anions were found to oxidize at potentials higher than other common anions used in electrochemical technologies. This feature makes these media highly desirable for use as electrolytes in electrochemical capacitors, and with the addition of, e.g., lithium Imide or lithium Methide, is an additional property making these media useful as electrolytes in rechargeable lithium-ion batteries.

Hydrophobic ionic liquids of the invention having both large cations and large anions have reduced ionic conductivity. However, the addition of a polar organic liquid as a cosolvent enhances the ionic conductivity by lowering the solution viscosity. An improved electrolyte of the invention, for use in an electrochemical cell, includes a hydrophobic ionic liquid as described above and a polar organic liquid, which, in one embodiment, is selected from the group consisting of linear ethers, cyclic ethers, esters, carbonates, lactones, nitriles, amides, sulfones and sulfolanes. In another embodiment, the polar organic liquid is selected from the group consisting of diethylether, dimethoxyethane, tetrahydrofuran, dioxane, dioxolane, methyltetrahydrofuran, methyl formate, ethyl formate, methyl propionate, propylene carbonate, ethylene carbonate, dimethyl carbonate, diethyl carbonate, ethylmethyl carbonate, dibutyl carbonate, butyrolactones, acetonitrile, benzonitrile, nitromethane, nitrobenzene, dimethylformamide, N-methylpyrolidone, dimethylsulfone, tetramethylene sulfone, sulfolane and thiophene.

Furthermore, the hydrophobic ionic liquids of the invention afford high thermochemical stability. The Imide anions decompose at temperatures beyond 300°C while imidazolium cations are stable to 270°C. Room temperature molten salts containing Lewis acids typically decompose below 150°C.

Large anions such as Imide are also known to influence the stereoselectivity of products derived from organic synthesis reactions. For example, Handy and coworkers (Synthetic Letters, 565, 1995) found that 4 M solutions of lithium Imide in acetone or diethyl ether are practical media for promoting and accelerating [4+2] cycloaddition reactions. An unexpected finding of this work was that Imide gave predominantly exo-adducts while the smaller perchlorate anion gave the expected endo-adduct. This suggests that the hydrophobic ionic liquids of the invention, which comprise large anions, may, in concentrations of from 4-5 M, be useful as synthetic media for reactions in which novel stereochemical outcomes are desirable.

### BRIEF DESCRIPTION OF THE DRAWINGS

A more complete appreciation of the invention may be obtained from a consideration of the following detailed description taken in conjunction with the drawings in which:
Figure 1 shows a series of linear sweep voltammograms acquired at a platinum electrode in several solvent-free ionic liquids; and
Figure 2 shows a plot of calculated anion ionization potentials vs. experimentally measured electrochemical oxidation potentials;
Figure 3 shows an arrangement of components of an electrochemical cell or battery utilizing a hydrophobic ionic liquid of the invention; and
Figure 4 shows an arrangement of components of a capacitor utilizing a hydrophobic ionic liquid of the invention.

### DETAILED DESCRIPTION

The following examples are presented to illustrate the advantages of the present invention and to assist one of ordinary skill in making and using the same. These examples are not intended in any way otherwise to limit the scope of the disclosure.

### EXAMPLE I (Comparative)

### Synthesis of 1,2-dimethyl-3-propylimidazolium Imide and Methide (Comparative)

1,2-Dimethyl-3-propylimidazolium Imide and Methide are prepared by a metathesis reaction in acetonitrile according to the following generic formula:

DMPICI + LiX → DMPIX + LiCI↓

where X=Imide (Im⁻) or Methide (Me⁻).

The starting compound 1,2-dimethyl-3-propylimididazolium chloride (DMPICl) was prepared and purified according to the method of Gifford and Palmisano (J. Electrochem. Soc. 134:610, 1987). LiIm (3M Company, Minneapolis, MN) was used without further purification. LiMe (mp 272-273°C) was prepared in accordance with U.S. Patent 5,273,840, hereby incorporated by reference, and was purified by dissolution in hot deionized H₂O followed by refluxing with activated charcoal. After filtration with Celite® filter aid, the H₂O was removed under vacuum, leaving pure LiMe.

All DMPIX reaction mixtures in acetonitrile were cold-vacuum filtered to remove LiCl. The acetonitrile was then removed under vacuum and the DMPIX residue taken up in CH₂Cl₂. This solution was extracted with deionized H₂O to remove residual LiCl. At this stage in the synthesis, a water soluble room temperature molten salt would not have remained in CH₂CH₂ when extracted with an aqueous layer. However, because DMPI Me is at least 1000 fold less soluble in water than the prior art compounds DMPI AsF₆ or DMPI PF₆, the extraction of residual precursor material was successful and the synthesis could proceed.

When the aqueous phase tested negative for Cl⁻ *via* an aqueous AgNO₃ solution, the organic phase was dried over MgSO₄, filtered, and the CH₂Cl₂ removed under reduced pressure. The residual salt was dried under vacuum at 80°C for 16 hours. If at this point the product was not a colorless oil, DMPIX was taken up in dry CH₂Cl₂ and refluxed with activated charcoal for 4 hours. After the addition of Celite® the mixture was vacuum filtered through a Whatman GF/F 0.7 µm filter disk and the solution was treated as before. All DMPIX yields exceeded 95%. Fourier transform infrared spectroscopy (FTIR) spectra of DMPIX were essentially identical to an overlay of the FTIR spectra of DMPICl and LiX.

### EXAMPLE II

### Synthesis of n-butylpyridinium Imide (BPIm) and Methide (BPMe) (Comparative)

Exemplary pyridinium ionic liquids, n-butylpyridinium Imide (BPIm) and Methide (BPMe), are also prepared by a metathesis reaction in acetonitrile, as described above, according to the formula:

BPCI + LiX → BPX + LiCI↓

where X = Im⁻ or Me⁻.

The starting compound n-butylpyridinium chloride (BPCI) was synthesized and purified according to the methods of J. Robinson and R.A. Osteryoung, as described in J. Chem. Soc. 101:323 (1979).

### EXAMPLE III

### Synthesis of 1-ethyl-3-methylimidazolium perfluoro-1,1-dimethylpropyl alkoxide (EMI pfDMP)

1-Ethyl-3-methylimidazolium perfluoro-1,1-dimethylpropyl alkoxide (EMI pfDMP) was prepared by a metathesis reaction in acetonitrile, as described above, according to the formula:

EMI CI + LiX → EMI X + LiCI↓

where X = pfDMP.

The starting compound EMI Cl was prepared in accordance with Wilkes et al., Inorg. Chem. 21:1263 (1982), and Li pfDMP was from the Department of Chemistry, Portland State University, Portland, OR (P. Nelson, G. Gard, in house work). The EMI pfDMP product was found to be a colorless oil, insoluble in water, with an FTIR spectrum which is essentially equivalent to an overlay of the FTIR spectra of the two starting materials, EMI Cl and Li pfDMP.

### EXAMPLE IV

### Synthesis of perfluoro-1-ethyl-3-methylimidazolium Imide (pfEMI Im)

Perfluoro-1-ethyl-3-methylimidazolium Imide (pfEMI Im) was synthesized, according to a newly developed procedure, from 1-ethyl-3-methylimidazolium Imide (EMI Im) and commercially available K₂NiF₆ in anhydrous HF (aHF) at -30°C in accordance with the generic formula:

EMI Im + 11 K₂NiF₆ → pfEMI Im + 22 KF + 11 NiF2↓ + 11 HF

Since one equivalent of K₂NiF₆ replaces one H with F on the EMI cation, and since there are eleven replaceable hydrogen atoms on EMI⁺, an amount of K₂NiF₆ equal to eleven times the amount of EMI, in equivalents, was used in this example in order to perfluorinate the cation. Should a lesser degree of fluorination be desirable, a correspondingly smaller amount of K₂NiF₆ may be used.

The starting compound EMI Im was synthesized in accordance with the method of Koch and coworkers (J. Electrochem. Soc., 142:L116, 1995). K₂NiF₆ (Ozark-Mahoning Atochem, Tulsa, OK) was used without further purification. The two reagents were placed in separate Teflon® tubes sealed at the bottom and inserted into a 3-way Teflon® PFA ¼" tubing union T. The third port of the union T was then connected to a vacuum line. aHF was condensed onto each reagent under reduced pressure at -30°C. EMI Im formed a clear, colorless solution with the aHF while K₂NiF₆ formed a deep purple solution with the aHF. When a small amount (∼ 10 v/o) of the K₂NiF₆ solution was transferred by tipping onto the EMI Im/aHF solution, an instantaneous reaction took place such that the purple color was immediately discharged and replaced with a yellow precipitate (NiF₂). Additional small increments of K₂NiF₆/aHF were added to the EMI Im/aHF solution until the reaction had gone to completion as evidenced by the complete absence of purple color.

The aHF was removed under vacuum after which the union T was disconnected from the vacuum line and the contents added to water into which the NiF₂ dissolved. The aqueous solution was extracted with ether, and when the ether was removed under vacuum, a clear colorless oil remained. The FTIR spectrum of this material was consistent with that of pfEMI Im, i.e., no C-H stretching or bending modes were observed in the spectrum while the bands associated with the Im⁻ anion were intact. These data indicate that all of the C-H bonds in EMI⁺ were replaced by C-F bonds. Because the pfEMI Im was insoluble in water, it was deemed to be a hydrophobic ionic liquid.

The new perfluorinated nitrogen based cation pfEMI⁺ may also be paired with other anions having van der Waals volumes less than 100 Å³ to produce an ionic liquid, or perhaps a solid at room temperature, having hydrophobic properties. As the synthetic fluorination technique disclosed herein is a general technique, all of the nitrogen-containing ring systems described in this application may be similarly perfluorinated.

### EXAMPLE V (Comparative)

### Resistance to electrochemical oxidation

Four compounds investigated by linear sweep voltammetry had a common cation, 1,2-dimethyl-3-propylimidazolium (DMPI), shown below.

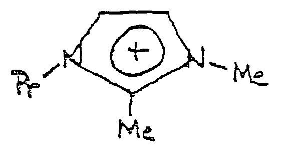

DMPI Im is an ionic liquid of the invention while DMPI PF₆ and DMPI AsF₆ are prior art compounds. Because DMPI PF₆ and DMPI AsF₆ were solids at room temperature, this experiment was conducted at 80°C to assure that all of the salts were fluid.

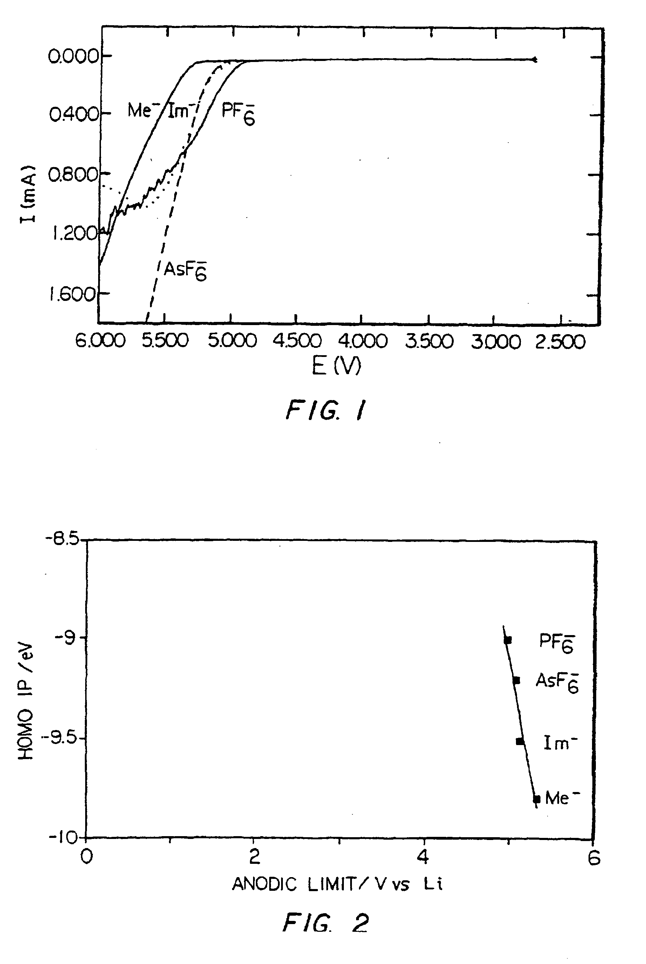

Figure 1 shows a series of linear sweep voltammograms acquired at a platinum electrode for each of the ionic liquids investigated. These overlays reveal that the order of oxidation is DMPI PF₆, DMPI AsF₆, DMPI Im and DMPI Me (Comparative). When the platinum electrode was scanned from the rest potential at 2.7V (vs. a Li reference) to more positive voltages, it was observed that DMPI PF₆ began to oxidize with the evolution of gas at 5.00V. Next, DMPI AsF₆ oxidized at 5.10V, followed by DMPI Im at 5.13V and DMPI Me at 5.35V. As assurance that this reactivity order was not an artifact of the platinum electrode, the same experiment was carried out on glassy carbon, and tungsten. All of the data are collected in Table 1 and show that, indeed, DMPI Im and DMPI Me, exemplary hydrophobic ionic liquids of the invention, are anodically robust. This feature is of critical importance in designing electrochemical generators that operate at high anodic potentials.

**Table 1:**

| Oxidation potentials of DMPIX salts at 1mA/cm². | | | |
|---|---|---|---|
| **Eₐ WORKING ELECTRODE ± 20 mV^{a}** | | | |
| **X** | **Glassy Carbon** | **W** | **Pt** |
| PF₆⁻ | 4.94 | 4.72 | 5.00 |
| AsF₆⁻ | 5.05 | 4.75 | 5.10 |
| Im⁻* | 5.06 | 5.16 | 5.13 |
| Me⁻* | 5.34 | 5.34 | 5.35 |

| | | | |
|---|---|---|---|
| ^{a} *vs*. Li⁺/Li at 80° C. | | | |
| * Comparative | | | |

Theoretical oxidation potentials were then calculated by an *ab initio* technique and plotted against the experimental data from the platinum electrode. Figure 2 shows a plot of calculated highest occupied molecular orbital (HOMO) anion ionization potential energies vs. experimental oxidation potentials determined at Pt for DMPIX, where X = AsF₆⁻, PF₆⁻, Im⁻, or Me⁻. A correlation coefficient of 0.91 was obtained, showing a good correlation of the theoretical and experimentally observed values. The calculated oxidation potentials or highest occupied molecular orbital (HOMO) ionization potentials for Imide and Methide are the largest values reported to date, indicating that these two anions are highly robust.

The room temperature oxidation potentials were also determined for DMPI Im and DMPI Me at a platinum electrode and were found to be 5.40 and 5.65V, respectively. These experimental values are among the highest reported to date for organic anions.

### EXAMPLE VI (Comparative)

### Ionic Conductivity

While the Im⁻ and Me⁻ anions manifest an extremely high resistance to electrochemical oxidation, their large size reduces the ionic conductivity of ionic liquids incorporating them. Table 2 compares the room temperature conductivities of eight different solvent-free ionic liquids as a function of three different cations and three different anions. As the volume of either the cation and/or anion increases, the specific conductance decreases. For example, the addition of one more alkyl group to EMI, forming DMPI, reduces the conductivity by a factor of 3 when the anion is held constant.

**Table 2:**

| Specific conductivities of various molten salts at 22°C. | | | | |
|---|---|---|---|---|
| **Molten Salt** | **σ, mScm⁻¹** | **V^{a}, A³** | **V^{b}, A³** | |
| | | | | |
| EMI AlCl₄* | | 15.0 | 118 | 113 |
| EMI Im* | | 8.3 | 118 | 144 |
| EMI Me* | | 1.3 | 118 | 206 |
| | | | | |
| DMPI AlCl₄* | | 7.1 | 152 | 114 |
| DMPI Im* | | 2.5 | 152 | 144 |
| DMPI Me* | | 0.5 | 152 | 206 |
| | | | | |
| BP^{c} AlCl₄* | | 10.3^{d} | 152 | 113 |
| BP Im | | 3.1 | 152 | 143 |

| | | | | |
|---|---|---|---|---|
| ^{a} van der Waals volume of the cations | | | | |
| ^{b} van der Waals volume of the anions | | | | |
| ^{c} n-butylpyridinium | | | | |
| ^{d} at 40°C | | | | |
| * Comparative | | | | |

The combination of a large DMPI⁺ cation and either the Im⁻ or Me⁻ anion greatly reduces the ionic conductivity. However, the addition of a polar organic liquid as a cosolvent to DMPIX enhances the ionic conductivity by lowering the solution viscosity. For example, 2M DMPIMe in 1:1 (v/v) of propylene carbonate (PC) and dimethyl carbonate (DMC) has a specific conductivity of 13 Mscm⁻¹, which is 26 times the conductivity of the neat melt. PC/DMC is a common cosolvent mixture used in Li batteries and electrochemical capacitors.

Table 3 collect the van der Waals volume data for a series of polyatomic anions used in electrochemical applications.

**Table 3:**

| van der Waals volumes of various anions. | | |
|---|---|---|
| **Anion** | **V^{a}, A³** | **V^{b}, A³** |
| | | |
| BF₄⁻ | 48 | 49 |
| ClO₄⁻ | 52 | 55 |
| PF₆⁻ | 68 | 69 |
| AsF₆⁻ | 73 | 73 |
| CF₃SO₃⁻ | 80 | 80 |
| (CF₃)₂PF₄⁻ | 105 | - |
| AlCl₄⁻ | 113 | - |
| (CF₂SO₃⁻)₂ | 124 | - |
| SF₅CF₂SO₃⁻ | 124 | - |
| (CF₃SO₂)₂N⁻ | 143 | 146 |
| CF₃CF₂(CF₃)₂CO⁻ | 144 | - |
| CF₃(CF₂)₃SO₃⁻ | 149 | - |
| (CF₃SO₂)₂CH⁻ | 149 | - |
| (CF₂CF₂SO₃⁻)₂ | 170 | - |
| (SF₅)₃C⁻* | 199 | - |
| (CF₃SO₂)₃C⁻* | 206 | - |
| [O(CF₃)₂C₂(CF₃)₂O]₂PO⁻ | 235 | - |
| CF₃(CF₂)₇SO₃⁻ | 249 | - |

| | | |
|---|---|---|
| ^{a} calculated via Hyperchem® software | | |
| ^{b} crystallographic data for comparison to the calculated values | | |
| * Comparative | | |

### EXAMPLE VII

### Use of hydrophobic ionic liquids of the invention in electrochemical cells or batteries

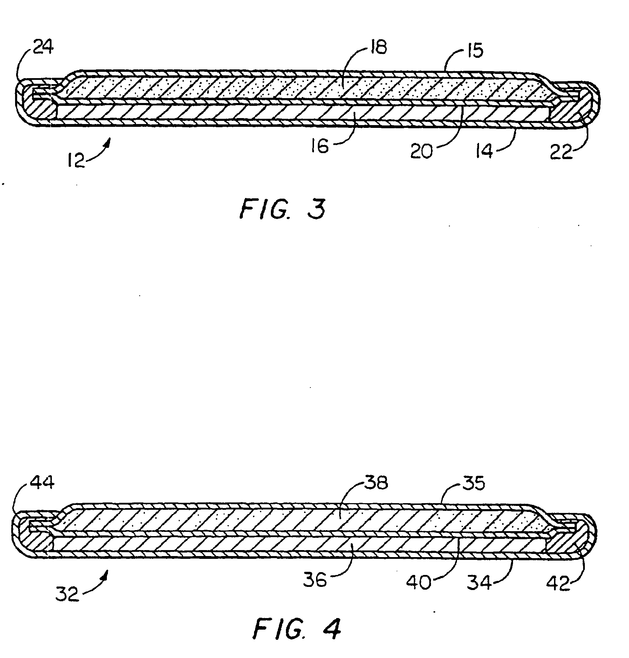

An electrochemical cell or battery of the invention includes as an electrolyte a hydrophobic ionic liquid of the invention. Referring to Fig. 3, such a cell 12 has, within a conductive container 14 and cover 15, an anode 16 and a cathode particle mix 18. A separator 20 which includes an electrolyte is placed between the anode and the mix. Container 14 is crimped at the edges 24 capturing cover 15 under an insulating gasket 22. Cells so formed may be configured for either parallel or series operation.

The electrolyte can include a polar cosolvent along with the hydrophobic ionic liquid, as described under Example IV, to enhance ionic conductivity. For lithium-ion batteries, the electrolyte will also include the dissolved salt LiX (where X = any anion of the invention).

### EXAMPLE VIII

### Use of hydrophobic ionic liquids of the invention in electrochemical capacitors

An electrochemical capacitor of the invention similarly includes a hydrophobic ionic liquid of the invention and is configured as shown in Figure 4. An electrochemical capacitor is an electrochemical storage device in which electric charge is stored in the electrical double-layer formed at the interface between a polarizable electrode and an electrolyte solution when dc voltage is applied.

Referring to Fig. 4, such a cell 32 has, within a conductive container 34 and cover 35, two electrodes 36 and 38 which may be composed of the same material or different materials. A separator 40 which includes an electrolyte is placed between the two electrodes. Container 34 is crimped at the edges 44, capturing cover 35 under an insulating gasket 42. Cells so formed may be configured for either parallel or series operation.

Such a capacitor was tested for its ability to withstand long-term cycling in the following experiment:

Two 3-layer Spectracarb® 2220 carbon cloth electrodes were assembled in union Ts containing EMI Im electrolyte and placed on battery cycling equipment for long-term cycling at a low current density. The current was set to 3 Ma/cm² with Cell A cycling from 0.2V to 2.0V and Cell B cycling from 0.2V to 3.0V.

The data obtained are summarized in Table 4 and show that the capacitance for both cells continued to remain above 25 F/g, with a specific capacitance of 100 F/g per electrode. Although Cell B displays slightly higher capacitance values for the 0.2 to 3.0V range, it has lost 15% of its initial capacitance over the 600 cycles, while Cell A's capacitance values for the 0.2 to 2.0V range have remained constant through 1100 cycles.

**Table 4:**

| Long-term cycling of Tee-cell capacitors having SC 2220 carbon cloth electrodes in EMI Im electrolyte | | | | | | | |
|---|---|---|---|---|---|---|---|
| Cell^{a} | Voltage Window (V) | Cycle # | Time (min.) | | Capacitance (F/g) | | |
| | | | C^{b} | D | C | D | D/C |
| A | 0.2-2.0 | 5 | 20.0 | 20.0 | 25.0 | 25.0 | 1.00 |
| | | 100 | 21.7 | 20.0 | 27.0 | 25.0 | 0.93 |
| | | 330 | 20.0 | 20.0 | 25.0 | 25.0 | 1.00 |
| | | 1100 | 20.0 | 19.3 | 25.0 | 24.1 | 0.96 |
| | | | | | | | |
| B | 0.2-3.0 | 10 | 39.0 | 37.7 | 31.3 | 30.0 | 0.97 |
| | | 140 | 36.0 | 35.0 | 28.9 | 28.1 | 0.97 |
| | | 605 | 33.3 | 33.3 | 26.7 | 26.3 | 0.98 |

| | | | | | | | |
|---|---|---|---|---|---|---|---|
| ^{a} each cell uses three 1 cm² carbon cloth discs (13 mg per disc) as an electrode Current = 3 Ma/cm², which is equivalent to 1 Ma/disk or 75 Ma/g | | | | | | | |
| ^{b} C = charge and D = discharge | | | | | | | |

## Claims

1. A hydrophobic ionic liquid comprising a cation and an anion, wherein said cation is selected from the group consisting of: 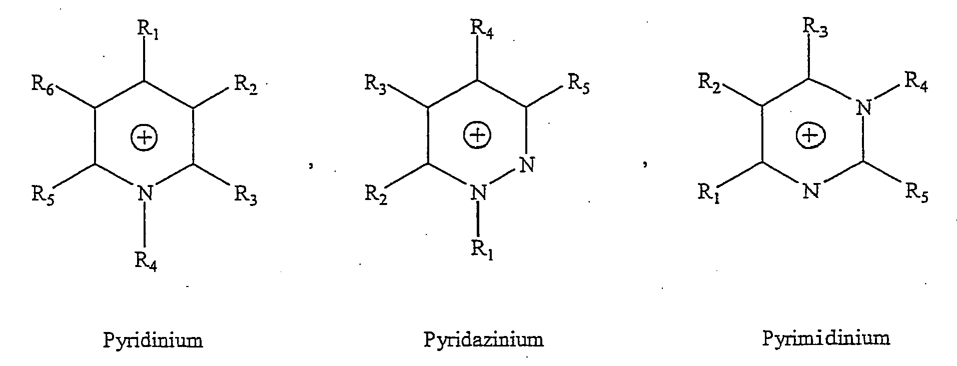 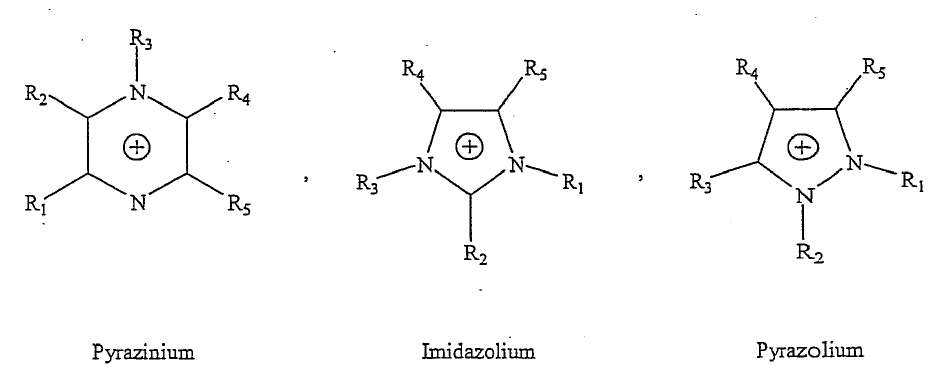 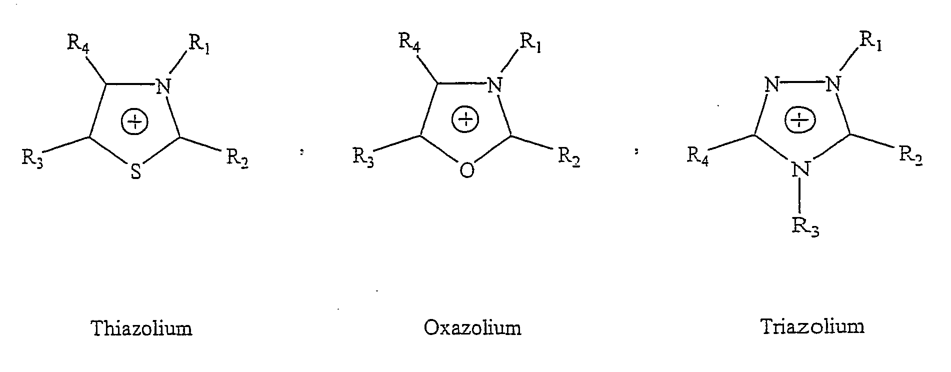 wherein R₁, R₂, R₃, R₄, R₅, and R₆ are either H; F; separate alkyl groups of from 1 to 4 carbon atoms, respectively, or joined together to constitute a unitary alkylene radical of from 2 to 4 carbon atoms forming a ring structure converging on N; or separate phenyl groups; and wherein the alkyl groups, alkylene radicals or phenyl groups may be substituted with electron withdrawing groups; and
wherein said anion is a non-Lewis acid-containing polyatomic anion having a van der Waals volume exceeding 100 Å³, with the proviso, that
a. compounds of the general formula 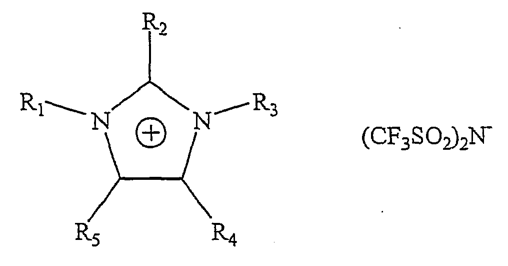 in which R₁ and R₃ are the same or different and each represent a straight or branched alkyl radical, a fluoroalkyl radical, or an alkoxyalkyl radical of 1 to 4 carbons, and R₂, R₄, and R₅ are the same or different and each represent a hydrogen atom or an alkyl radical with 1 to 3 carbon atoms,
b. the compounds N-methylpyridinium perfluorobutylsulfonate, N-methylpyridinium perfluorooctanesulfonate and the N,N-dimethylimidazolium perfluorobutylsulfonate and
c. methide salts are excluded.

2. The hydrophobic ionic liquid of claim 1 wherein, in said cation, a substituent electron withdrawing group is selected from the group consisting of F-, Cl-, CF₃-, SF₅-, CF₃S-, (CF₃)₂CHS- and (CF₃)₃CS-.

3. The hydrophobic ionic liquid of claim 1 wherein said cation has the formula 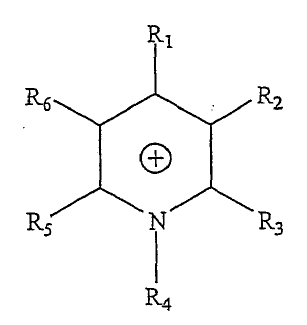 wherein R₁, R₂, R₃, R₄, R₅, and R₆ are either H; F; separate alkyl groups of from 1 to 4 carbon atoms, respectively, or joined together to constitute a unitary alkylene radical of from 2 to 4 carbon atoms forming a ring structure converging on N; or separate phenyl groups; and wherein the alkyl groups, alkylene radicals or phenyl groups may be substituted with electron withdrawing groups.

4. The hydrophobic ionic liquid of claim 1 wherein said cation has the formula 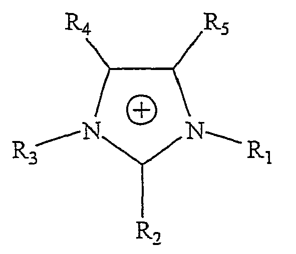 wherein R₁, R₂, R₃, R₄ and R₅ are either H; F; separate alkyl groups of from 1 to 4 carbon atoms, respectively, or joined together to constitute a unitary alkylene radical of from 2 to 4 carbon atoms forming a ring structure converging on N; or separate phenyl groups; and wherein the alkyl groups, alkylene radicals or phenyl groups may be substituted with electron withdrawing groups.

5. The hydrophobic ionic liquid of claim 1 wherein said cation has the formula 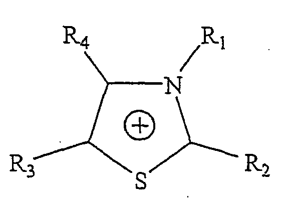 wherein R₁, R₂, R₃, R₄ and R₅ are either H; F; separate alkyl groups of from 1 to 4 carbon atoms, respectively, or joined together to constitute a unitary alkylene radical of from 2 to 4 carbon atoms forming a ring structure converging on N; or separate phenyl groups; and wherein the alkyl groups, alkylene radicals or phenyl groups may be substituted with electron withdrawing groups.

6. The hydrophobic ionic liquid of claim 1 wherein said cation has the formula 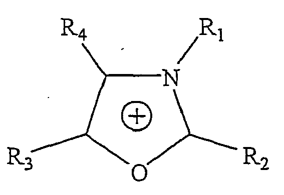 wherein R₁, R₂, R₃, R₄ and R₅ are either H; F; separate alkyl groups of from 1 to 4 carbon atoms, respectively, or joined together to constitute a unitary alkylene radical of from 2 to 4 carbon atoms forming a ring structure converging on N; or separate phenyl groups; and wherein the alkyl groups, alkylene radicals or phenyl groups may be substituted with electron withdrawing groups.

7. The hydrophobic ionic liquid of claim 1 wherein said anion has the formula 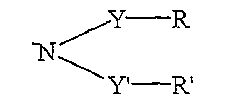 wherein each of the moieties Y and Y' are -SO₂- or -CO-; and the groups R and R' are separate halogenated alkyl groups of 1-4 carbon atoms.

8. The hydrophobic ionic liquid of claim 1 wherein said anion is perfluoro-1,1-dimethylpropyl alkoxide, having the formula CF₃CF₂(CF₃)₂CO-.

9. The hydrophobic ionic liquid of claim 1 wherein said anion is selected from the group consisting (CF₃)₂PF₄⁻,(CF₃)₃PF₃⁻(CF₃)₄PF₂⁻, (CF₃)₅PF⁻(CF₃)₆P⁻, SF₅CF₂SO₃⁻, SF₅CHFCF₂SO₃⁻, and [O(CF₃)₂C₂(CF₃)₂O]₂PO.

10. The hydrophobic ionic liquid of claim 1 wherein said anion is a mono- or di-perfluorosulfonate.

11. An electrolyte for use in an electrochemical cell comprising a hydrophobic ionic liquid comprising a cation and an anion, wherein said cation is selected from the group consisting of: 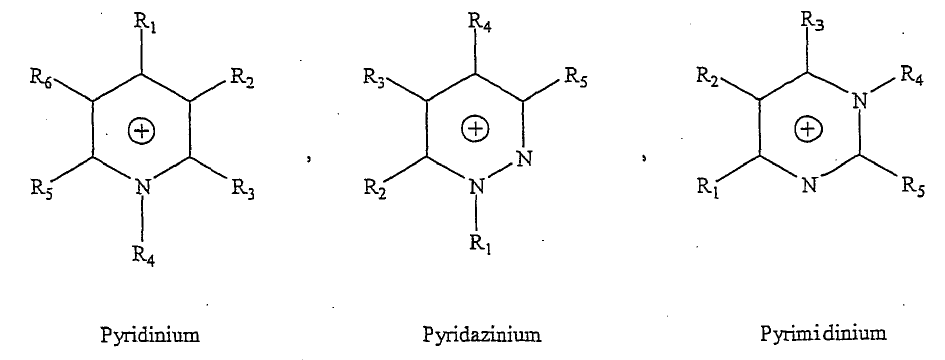 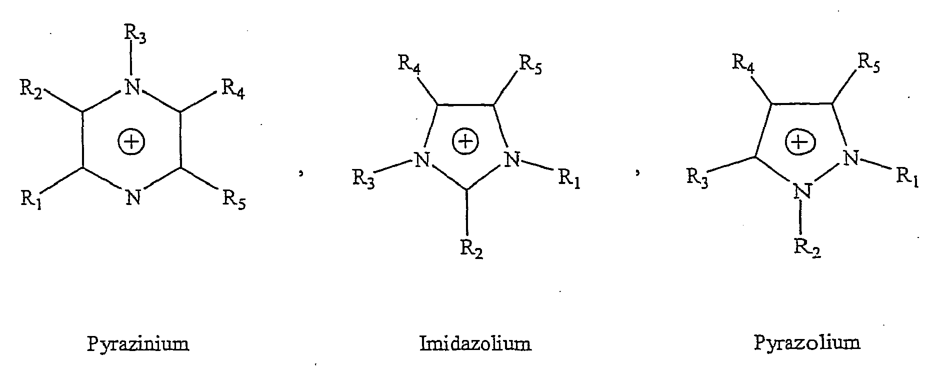 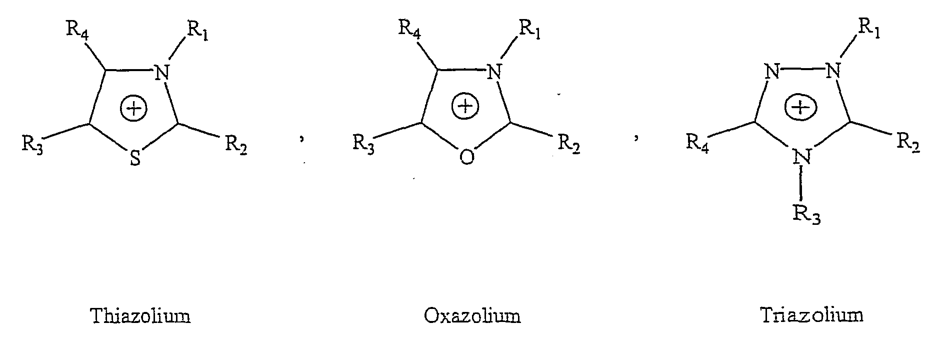 wherein R₁, R₂, R₃, R₄, R₅, and R₆ are either H; F; separate alkyl groups of from 1 to 4 carbon atoms, respectively, or joined together to constitute a unitary alkylene radical of from 2 to 4 carbon atoms forming a ring structure converging on N; or separate phenyl groups; and wherein the alkyl groups, alkylene radicals or phenyl groups may be substituted with electron withdrawing groups; and
wherein said anion is a non-Lewis acid-containing polyatomic anion having a van der Waals volume exceeding 100 Å³; and
a polar organic liquid, with the proviso that
a. solutions of compounds of the general formula 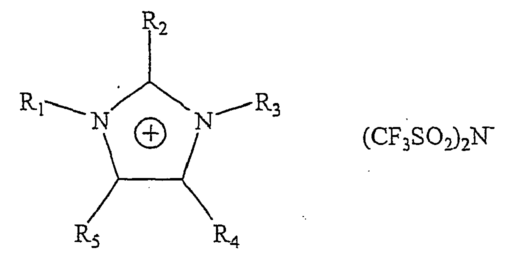 in which R₁ and R₃ are the same or different and each represent a straight or branched alkyl radical, a fluoroalkyl radical, or an alkoxyalkyl radical of 1 to 4 carbons, and R₂, R₄, and R₅ are the same or different and each represent a hydrogen atom or an alkyl radical with 1 to 3 carbon atoms, in an aprotic solvent,
b. solutions of compounds represented by the formula (1/nM)⁺[(ZY)₂N]⁻, (1/nM)⁺[(ZY)₃C]⁻, (1/nM(ZY)⁺[(ZY)₂CQ]⁻, wherein M represents a cation selected from pyridines and imidazoles, Y represents SO₂ or POZ, Q represents H, COZ or Z and each substituent Z represents independently a fluorine atom, or an organic moiety which may or may not be perfluorinated, which moiety optionally contains at least one polymerizable function, with the proviso that at least one of the Z-substituents represents a fluorine atom, in an aprotic solvent, and
c. methide salts are excluded.

12. The electrolyte of claim 11, wherein said polar organic liquid is selected from the group consisting of linear ethers, cyclic ethers, esters, carbonates, lactones, nitriles, amides, sulfones and sulfolanes.

13. The electrolyte of claim 11, wherein said polar organic liquid is selected from the group consisting of diethylether, dimethoxyethane, tetrahydrofuran, dioxane, dioxolane, methyltetrahydrofuran, methyl formate, ethyl formate, methyl propionate, propylene carbonate, ethylene carbonate, dimethyl carbonate, diethyl carbonate, ethylmethyl carbonate, dibutyl carbonate, butyrolactones, acetonitrile, benzonitrile, nitromethane, nitrobenzene, dimethylformamide, N-methylpyrolidone, dimethylsulfone, tetramethylene sulfone, sulfolane and thiophene.

14. An electrochemical cell comprising
an anode;
a cathode; and
an electrolyte comprising a hydrophobic ionic liquid comprising a cation and an anion, wherein said cation is selected from the group consisting of: 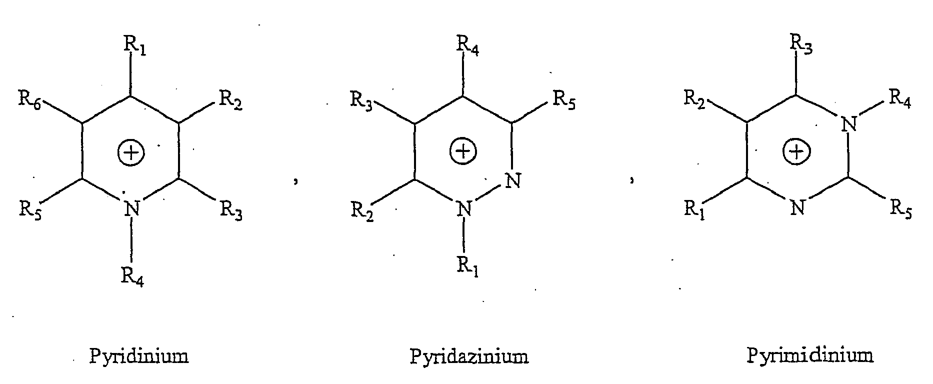 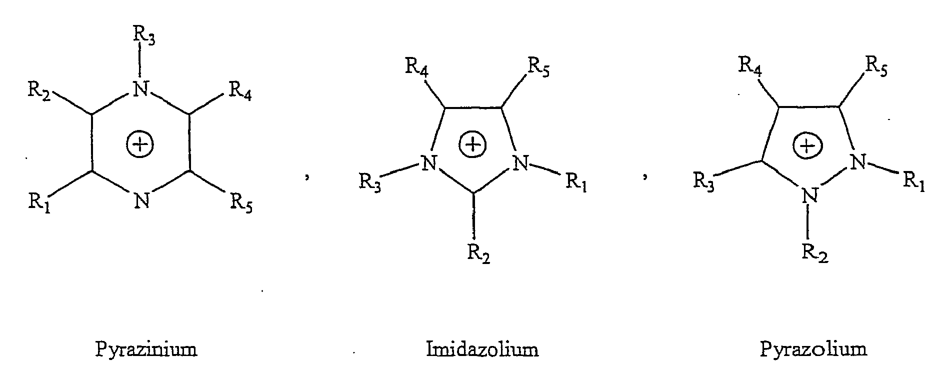 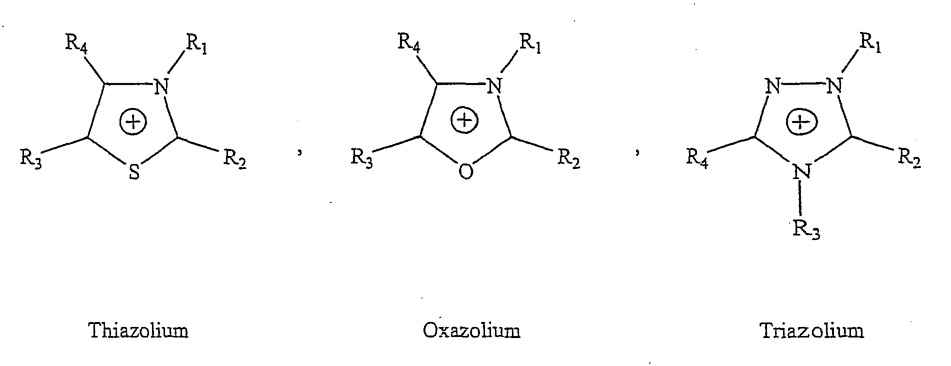 wherein R₁, R₂, R₃, R₄, R₅, and R₆ are either H; F; separate alkyl groups of from 1 to 4 carbon atoms, respectively, or joined together to constitute a unitary alkylene radical of from 2 to 4 carbon atoms forming a ring structure converging on N; or separate phenyl groups; and wherein the alkyl groups, alkylene radicals or phenyl groups may be substituted with electron withdrawing groups; and
wherein said anion is a non-Lewis acid-containing polyatomic anion having a van der Waals volume exceeding 100 Å³, with the proviso that
a. compounds of the general formula 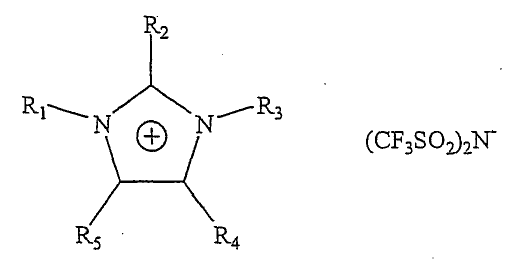 in which R₁ and R₃ are the same or different and each represent a straight or branched alkyl radical, a fluoroalkyl radical, or an alkoxyalkyl radical of 1 to 4 carbons, and R₂, R₄, and R₅ are the same or different and each represent a hydrogen atom or an alkyl radical with 1 to 3 carbon atoms,
b. solutions of compounds represented by the formula (1/nM)⁺[(ZY)₂N]⁻, (1/nM)⁺[(ZY)₃C]⁻, (1/nM)⁺[(ZY]₂CQ]⁻, wherein M represents a cation selected from pyridines and imidazoles, Y represents SO₂ or POZ, Q represents H, COZ or Z and each substituent Z represents independently a fluorine atom, or an organic moiety which may or may not be perfluorinated, which moiety optionally contains at least one polymerizable function, with the proviso that at least one of the Z-substituents represents a fluorine atom, in an aprotic solvent, and
c. methide salts are excluded.

15. An electrochemical cell comprising
an anode;
a cathode; and
an electrolyte comprising a hydrophobic ionic liquid comprising a cation and an anion, wherein said cation is selected from the group consisting of: 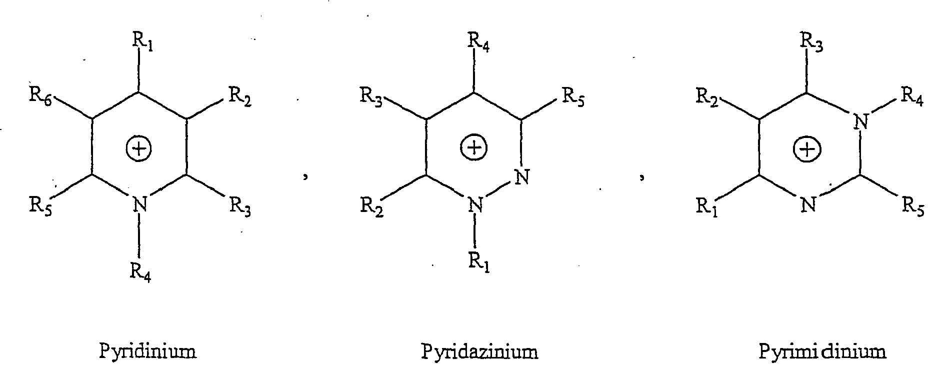 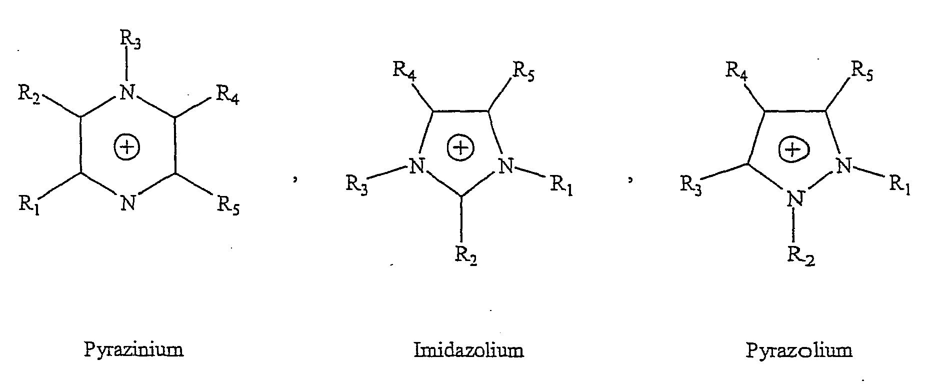 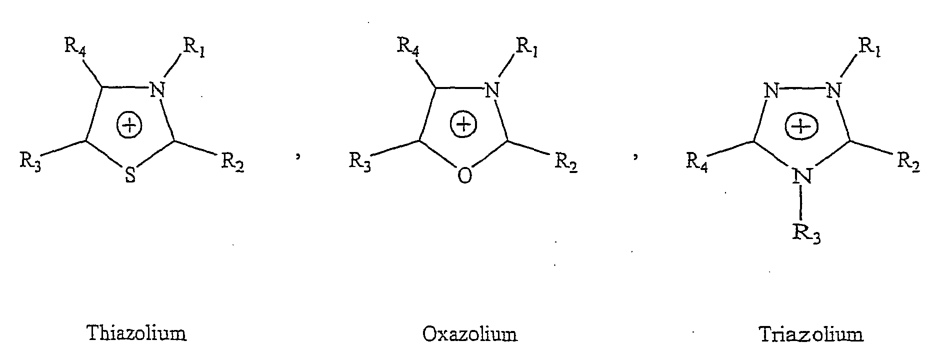 wherein R₁, R₂, R₃, R₄, R₅, and R₆ are either H; F; separate alkyl groups of hom 1 to 4 carbon atoms, respectively, or joined together to constitute a unitary alkylene radical of from 2 to 4 carbon atoms forming a ring structure converging on N; or separate phenyl groups; and wherein the alkyl groups, alkylene radicals or phenyl groups may be substituted with electron withdrawing groups; and
wherein said anion is a non-Lewis acid-containing polyatomic anion having a van der walls volume exceeding 100 Å³,
- and a lithium salt.

16. The electrochemical cell of claim 14 or claim 15, wherein said electrolyte further comprises a polar organic liquid.

17. A capacitor comprising
a first electrode;
a second electrode; and
an electrolyte, said electrolyte comprising a hydrophobic ionic liquid comprising a cation and an anion, wherein said cation is selected from the group consisting of: 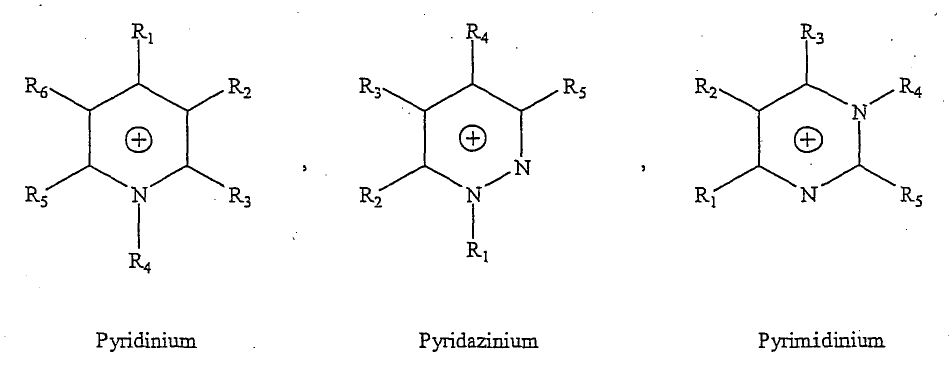 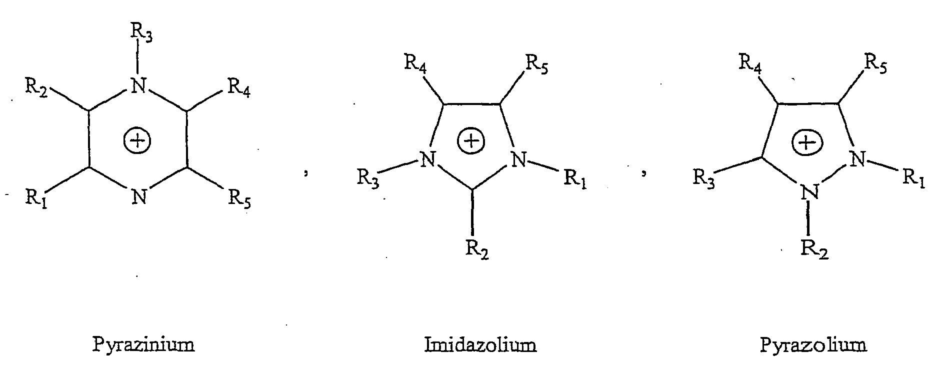 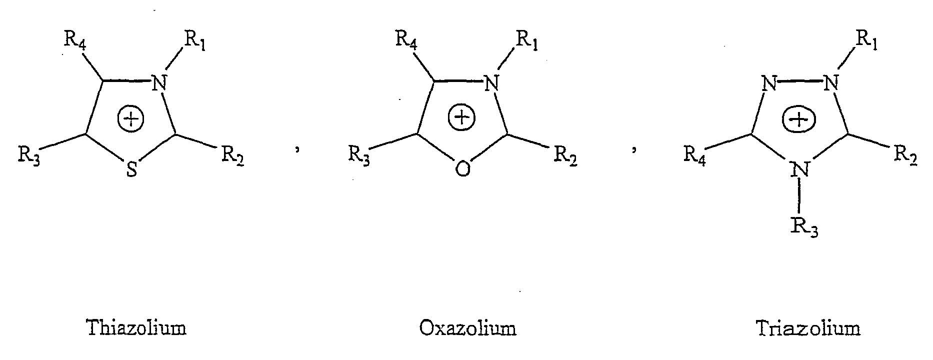 wherein R₁, R₂, R₃, R₄, R₅, and R₆ are either H; F; separate alkyl groups of from 1 to 4 carbon atoms, respectively, or joined together to constitute a unitary alkylene radical of from 2 to 4 carbon atoms forming a ring structure converging on N; or separate phenyl groups; and wherein the alkyl groups, alkylene radicals or phenyl groups may be substituted with electron withdrawing groups; and
wherein said anion is a non-Lewis acid-containing polyatomic anion having a van der Waals volume exceeding 100 Å³, with the proviso that solutions of compounds represented by the formula (1/nM⁺[(ZY)₂N]⁻ , (1/nM)⁺[(ZY)₃C]⁻, (1/nM)⁺[(ZY)₂CQ]⁻, wherein M represents a cation selected from pyridines and imidazoles, Y represents SO₂ or POZ, Q represents H, COZ or Z and each substituent Z represents independently a fluorine atom, or an organic moiety which may or may not be perfluorinated, which moiety optionally contains at least one polymerizable function, with the proviso that at least one of the Z-substituents represents a fluorine atom, in an aprotic solvent, and methide salts are excluded.

18. The hydrophobic ionic liquid according to claims 1-10, comprising a cation and an anion, wherein said cation is a perfluorinated, unsaturated, nitrogen-containing heterocycle and said anion is organic or inorganic.

## Patentansprüche

1. Hydrophobe ionische Flüssigkeit, umfassend ein Kation und ein Anion, wobei das Kation aus der Gruppe, bestehend aus: 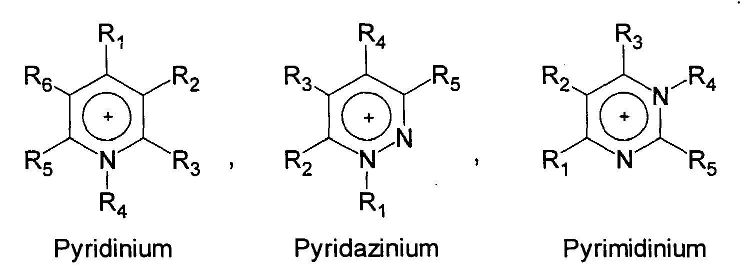 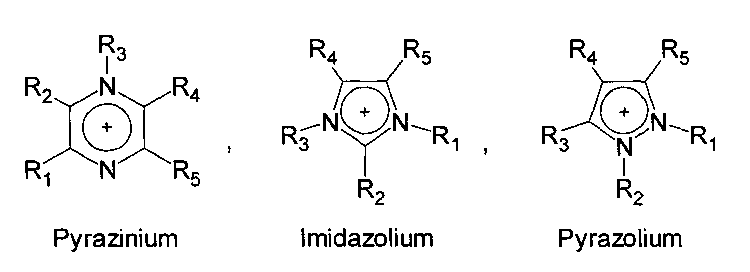 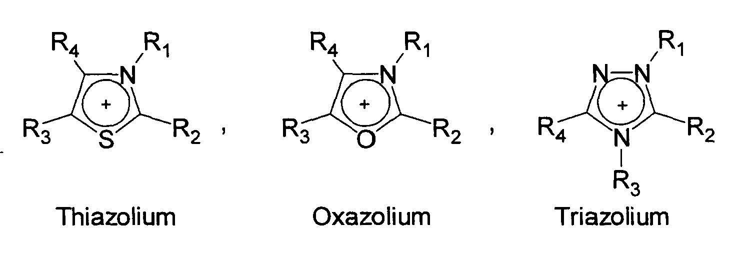 ausgewählt ist, worin R₁, R₂, R₃, R₄, R₅ und R₆ entweder H, F, jeweils getrennte Alkylgruppen mit 1 bis 4 Kohlenstoffatomen oder miteinander verbunden sind, um einen einheitlichen Alkylenrest mit 2 bis 4 Kohlenstoffatomen unter Bildung einer Ringstruktur, die am N zusammenläuft, zu bilden, oder getrennte Phenylgruppen sind, und
wobei die Alkylgruppen, Alkylenreste oder Phenylreste mit elektronenziehenden Gruppen substituiert sein können, und
wobei das Anion ein Nicht-Lewissäure-enthaltendes polyatomares Anion mit einem van-der-Waals-Volumen ist, das 100 A³ übersteigt, vorausgesetzt, dass
(a) Verbindungen der allgemeinen Formel 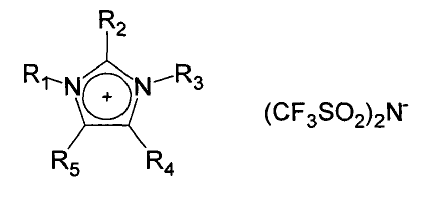 worin R₁ und R₃ gleich oder verschieden sind und jeweils einen geradkettigen oder verzweigten Alkylrest, einen Fluoralkylrest oder einen Alkoxyalkylrest mit 1 bis 4 Kohlenstoffatomen darstellen, und R₂, R₄ und R₅ gleich oder verschieden sind und jeweils ein Wasserstoffatom oder einen Alkylrest mit 1 bis 3 Kohlenstoffatomen darstellen,
(b) die Verbindungen N-Methylpyridiniumperfluorbutylsulfonat, N-Methylpyridiniumperfluoroctansulfonat und N,N-Dimethylimidazoliumperfluorbutylsulfonat und
(c) Methidsalze
ausgenommen sind.

2. Hydrophobe ionische Flüssigkeit nach Anspruch 1, wobei in dem Kation eine elektronenziehende Gruppe als Substituent aus der Gruppe, bestehend aus F-, Cl-, CF₃-, SF₅-, CF₃S-, (CF₃)₂CHS- und (CF₃)₃CS-, ausgewählt ist.

3. Hydrophobe ionische Flüssigkeit nach Anspruch 1, wobei das Kation die Formel 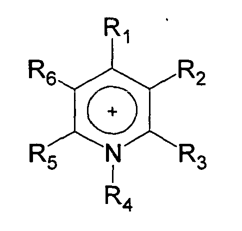 aufweist, worin R₁, R₂, R₃, R₄, R₅ und R₆ entweder H, F, jeweils getrennte Alkylgruppen mit 1 bis 4 Kohlenstoffatomen oder miteinander verbunden sind, um einen einheitlichen Alkylenrest mit 2 bis 4 Kohlenstoffatomen unter Bildung einer Ringstruktur, die am N zusammenläuft, zu bilden, oder getrennte Phenylgruppen sind, und wobei die Alkylgruppen, Alkylenreste oder Phenylgruppen mit elektronenziehenden Gruppen substituiert sein können.

4. Hydrophobe ionische Flüssigkeit nach Anspruch 1, wobei das Kation die Formel 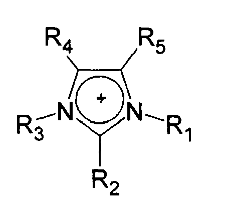 aufweist, worin R₁, R₂, R₃, R₄ und R₅ entweder H, F, jeweils getrennte Alkylgruppen mit 1 bis 4 Kohlenstoffatomen oder miteinander verbunden sind, um einen einheitlichen Alkylenrest mit 2 bis 4 Kohlenstoffatomen unter Bildung einer Ringstruktur, die am N zusammenläuft, zu bilden, oder getrennte Phenylgruppen sind, und wobei die Alkylgruppen, Alkylenreste oder Phenylgruppen mit elektronenziehenden Gruppen substituiert sein können.

5. Hydrophobe ionische Flüssigkeit nach Anspruch 1, wobei das Kation die Formel 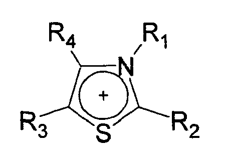 aufweist, worin R₁, R₂, R₃, R₄ und R₅ entweder H, F, jeweils getrennte Alkylgruppen mit 1 bis 4 Kohlenstoffatomen oder miteinander verbunden sind, um einen einheitlichen Alkylenrest mit 2 bis 4 Kohlenstoffatomen unter Bildung einer Ringstruktur, die am N zusammenläuft, zu bilden, oder getrennte Phenylgruppen sind, und wobei die Alkylgruppen, Alkylenreste oder Phenylgruppen mit elektronenziehenden Gruppen substituiert sein können.

6. Hydrophobe ionische Flüssigkeit nach Anspruch 1, wobei das Kation die Formel 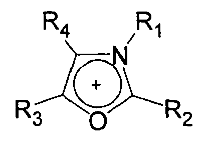 aufweist, worin R₁, R₂, R₃, R₄ und R₅ entweder H, F, jeweils getrennte Alkylgruppen mit 1 bis 4 Kohlenstoffatomen oder miteinander verbunden sind, um einen einheitlichen Alkylenrest mit 2 bis 4 Kohlenstoffatomen unter Bildung einer Ringstruktur, die am N zusammenläuft, zu bilden, oder getrennte Phenylgruppen sind, und wobei die Alkylgruppen, Alkylenreste oder Phenylgruppen mit elektronenziehenden Gruppen substituiert sein können.

7. Hydrophobe ionische Flüssigkeit nach Anspruch 1, wobei das Anion die Formel 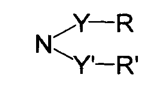 aufweist, worin jede der Einheiten Y und Y' -SO₂- oder -CO- ist und die Gruppen R und R' getrennte halogenierte Alkylgruppen mit 1 bis 4 Kohlenstoffatomen sind.

8. Hydrophobe ionische Flüssigkeit nach Anspruch 1, wobei das Anion ein Perfluor-1,1-dimethylpropylalkoxid mit der Formel CF₃CF₂(CF₃)₂CO⁻ ist.

9. Hydrophobe ionische Flüssigkeit nach Anspruch 1, wobei das Anion aus der Gruppe, bestehend aus (CF₃)₂PF₄⁻, (CF₃)₃PF₃⁻, (CF₃)₄PF₂⁻, (CF₃)₅PF-, (CF₃)₆P⁻, SF₅CF₂SO₃⁻, SF₅CHFCF₂SO₃⁻ und [O(CF₃)₂C₂(CF₃)₂O]₂PO⁻, ausgewählt ist.

10. Hydrophobe ionische Flüssigkeit nach Anspruch 1, wobei das Anion ein Mono- oder Diperfluorsulfonat ist.

11. Elektrolyt zur Verwendung in einer elektrochemischen Zelle, umfassend eine hydrophobe ionische Flüssigkeit, die ein Kation und ein Anion umfasst, wobei das Kation aus der Gruppe, bestehend aus 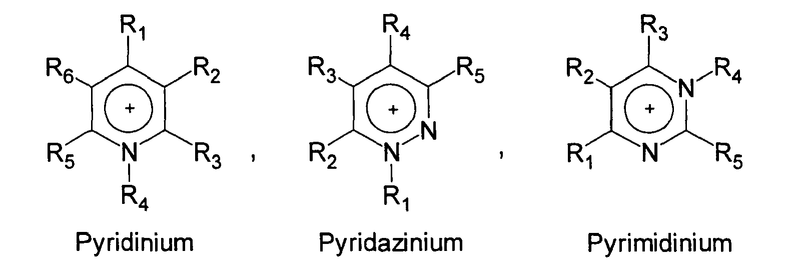 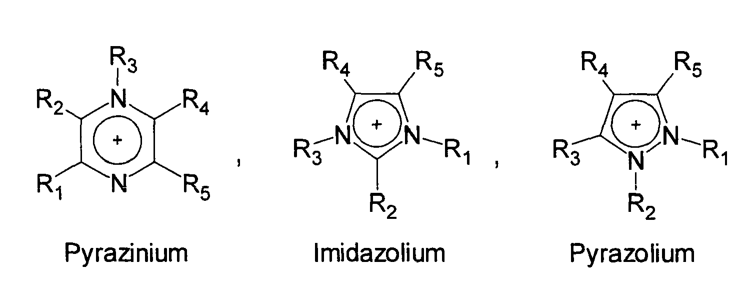 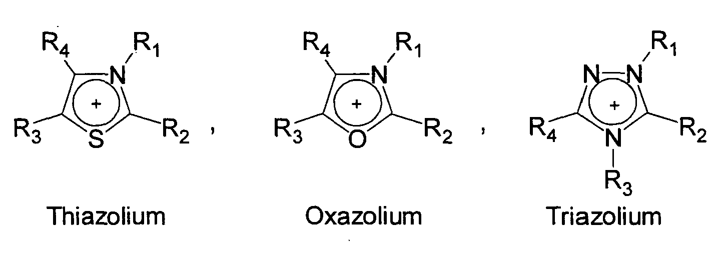 ausgewählt ist, worin R₁, R₂, R₃, R₄, R₅ und R₆ entweder H, F, jeweils getrennte Alkylgruppen mit 1 bis 4 Kohlenstoffatomen oder miteinander verbunden sind, um einen einheitlichen Alkylenrest mit 2 bis 4 Kohlenstoffatomen unter Bildung einer Ringstruktur, die am N zusammenläuft, zu bilden, oder getrennte Phenylgruppen sind, und wobei die Alkylgruppen, Alkylenreste oder Phenylgruppen mit elektronenziehenden Gruppen substituiert sein können, und
wobei das Anion ein Nicht-Lewissäure-enthaltendes polyatomares Anion mit einem van-der-Waals-Volumen ist, das 100 A³ übersteigt, und eine polare organische Flüssigkeit, vorausgesetzt, dass
(a) Lösungen von Verbindungen der allgemeinen Formel 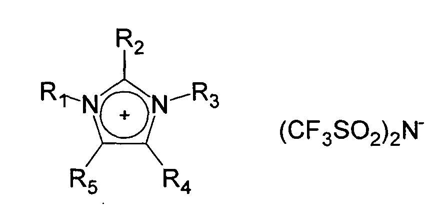 worin R₁ und R₃ gleich oder verschieden sind und jeweils einen geradkettigen oder verzweigten Alkylrest, einen Fluoralkylrest oder einen Alkoxyalkylrest mit 1 bis 4 Kohlenstoffatomen darstellen, und R₂, R₄ und R₅ gleich oder verschieden sind und jeweils ein Wasserstoffatom oder einen Alkylrest mit 1 bis 3 Kohlenstoffatomen darstellen, in einem aprotischen Lösungsmittel,
(b) Lösungen von Verbindungen, die durch die Formel (1/nM)⁺(ZY)₂N]⁻, (1/nM)⁺(ZY)₃C]⁻, (1/nM)⁺(ZY)₂CQ]⁻ dargestellt werden, worin M ein Kation, ausgewählt aus Pyridinen und Imidazolen, darstellt, Y SO₂ oder POZ darstellt, Q H, COZ oder Z darstellt, und jeder Substituent Z unabhängig ein Fluoratom oder eine organische Einheit darstellt, die perfluoriert sein kann oder nicht, wobei die Einheit optional zumindest eine polymerisierbare Funktion enthält, vorausgesetzt, dass zumindest einer der Z-Substituenten ein Fluoratom darstellt, in einem aprotischen Lösungsmittel, und
(c) Methidsalze
ausgenommen sind.

12. Elektrolyt nach Anspruch 1, wobei die polare organische Flüssigkeit aus der Gruppe, bestehend aus linearen Ethern, cyclischen Ethern, Estern, Carbonaten, Lactonen, Nitrilen, Amiden, Sulfonen und Sulfolanen, ausgewählt ist.

13. Elektrolyt nach Anspruch 1, wobei die polare organische Flüssigkeit aus der Gruppe, bestehend aus Diethylether, Dimethoxyethan, Tetrahydrofuran, Dioxan, Dioxolan, Methyltetrahydrofuran, Methylformiat, Ethylformiat, Methylpropionat, Propylencarbonat, Ethylencarbonat, Dimethylcarbonat, Diethylcarbonat, Ethylmethylcarbonat, Dibutylcarbonat, Butyrolactonen, Acetonitril, Benzonitril, Nitromethan, Nitrobenzol, Dimethylformamid, N-Methylpyrrolidon, Dimethylsulfon, Tetramethylensulfon, Sulfolan und Thiophen, ausgewählt ist.

14. Elektrochemische Zelle, umfassend
eine Anode;
eine Kathode; und
einen Elektrolyten, umfassend eine hydrophobe ionische Flüssigkeit, die ein Kation und ein Anion umfasst, wobei das Kation aus der Gruppe, bestehend aus 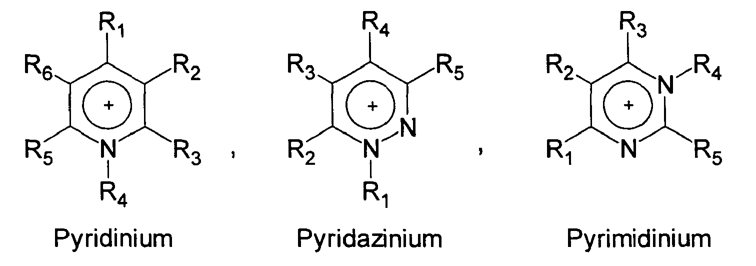 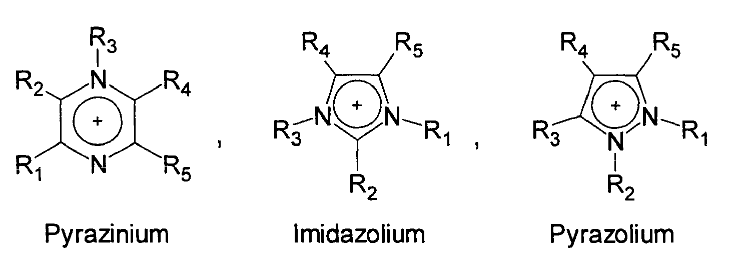 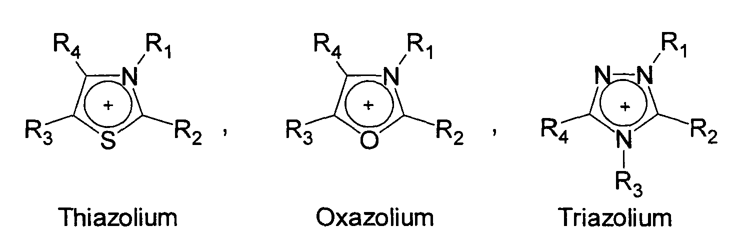 ausgewählt ist, worin R₁, R₂, R₃, R₄, R₅ und R₆ entweder H, F, jeweils getrennte Alkylgruppen mit 1 bis 4 Kohlenstoffatomen oder miteinander verbunden sind, um einen einheitlichen Alkylenrest mit 2 bis 4 Kohlenstoffatomen unter Bildung einer Ringstruktur, die am N zusammenläuft, zu bilden, oder getrennte Phenylgruppen sind, und wobei die Alkylgruppen, Alkylenreste oder Phenylgruppen mit elektronenziehenden Gruppen substituiert sein können, und
wobei das Anion ein Nicht-Lewissäure-enthaltendes polyatomares Anion mit einem van-der-Waals-Volumen ist, das 100 A³ übersteigt; vorausgesetzt, dass
(a) Verbindungen der allgemeinen Formel 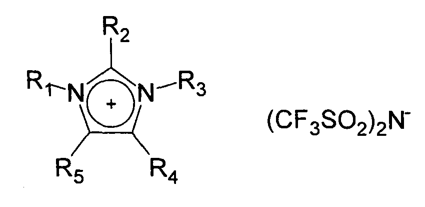 worin R₁ und R₃ gleich oder verschieden sind und jeweils einen geradkettigen oder verzweigten Alkylrest, einen Fluoralkylrest oder einen Alkoxyalkylrest mit 1 bis 4 Kohlenstoffatomen darstellen, und R₂, R₄ und R₅ gleich oder verschieden sind und jeweils ein Wasserstoffatom oder einen Alkylrest mit 1 bis 3 Kohlenstoffatomen darstellen,
(b) Lösungen von Verbindungen, die durch die Formel (1/nM)⁺(ZY)₂N]⁻, (1/nM)⁺(ZY)₃C]⁻, (1/nM)⁺(ZY)₂CQ]⁻ dargestellt werden, worin M ein Kation, ausgewählt aus Pyridinen und Imidazolen, darstellt, Y SO₂ oder POZ darstellt, Q H, COZ oder Z darstellt, und jeder Substituent Z unabhängig ein Fluoratom oder eine organische Einheit darstellt, die perfluoriert sein kann oder nicht, wobei die Einheit optional zumindest eine polymerisierbare Funktion enthält, vorausgesetzt, dass zumindest einer der Z-Substituenten ein Fluoratom darstellt, in einem aprotischen Lösungsmittel, und
(c) Methidsalze
ausgenommen sind.

15. Elektrochemische Zelle, umfassend
eine Anode;
eine Kathode; und
einen Elektrolyten, umfassend eine hydrophobe ionische Flüssigkeit, die ein Kation und ein Anion umfasst, wobei das Kation aus der Gruppe, bestehend aus 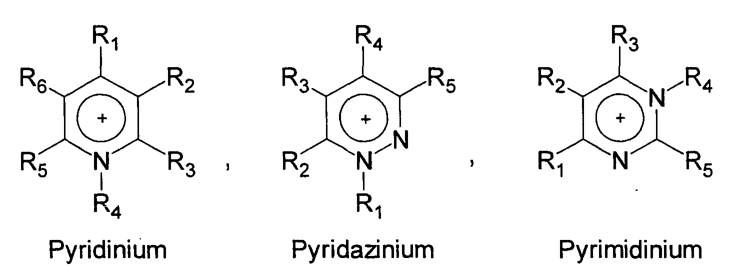 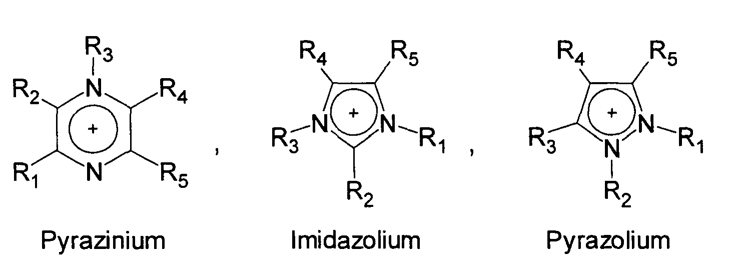 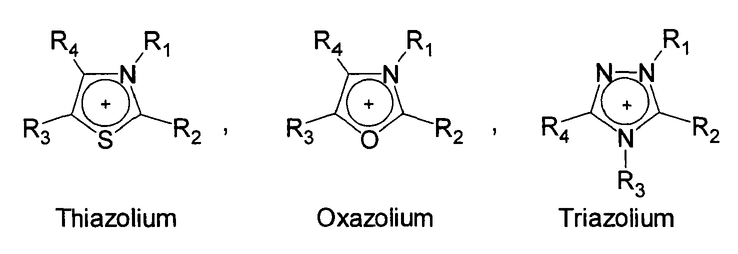 ausgewählt ist, worin R₁, R₂, R₃, R₄, R₅ und R₆ entweder H, F, jeweils getrennte Alkylgruppen mit 1 bis 4 Kohlenstoffatomen oder miteinander verbunden sind, um einen einheitlichen Alkylenrest mit 2 bis 4 Kohlenstoffatomen unter Bildung einer Ringstruktur, die am N zusammenläuft, zu bilden, oder getrennte Phenylgruppen sind, und wobei die Alkylgruppen, Alkylenreste oder Phenylgruppen mit elektronenziehenden Gruppen substituiert sein können; und
wobei das Anion ein Nicht-Lewissäure-enthaltendes polyatomares Anion mit einem van-der-Waals-Volumen ist, das 100 A³ übersteigt, und
ein Lithiumsalz.

16. Elektrochemische Zelle nach Anspruch 14 oder 15, wobei der Elektrolyt ferner eine polare organische Flüssigkeit umfasst.

17. Kondensator, umfassend
eine erste Elektrode;
eine zweite Elektrode; und
einen Elektrolyten, wobei der Elektrolyt eine hydrophobe ionische Flüssigkeit umfasst, die ein Kation und ein Anion umfasst, wobei das Kation aus der Gruppe, bestehend aus 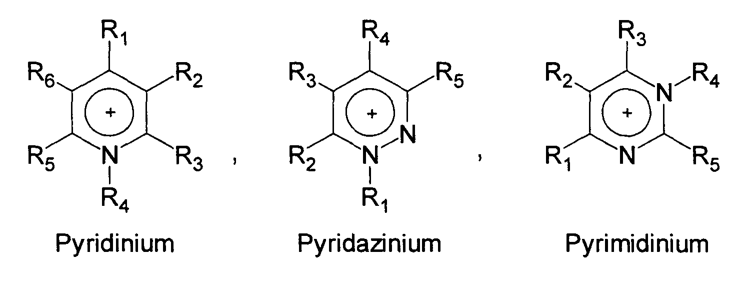 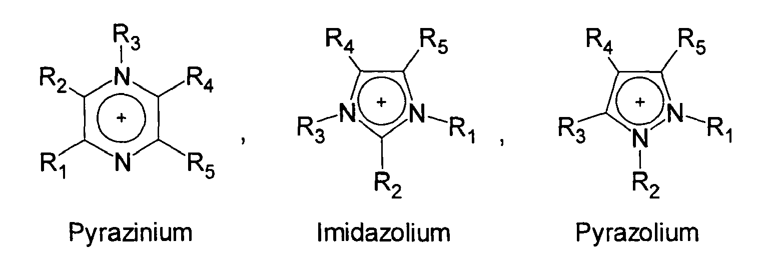 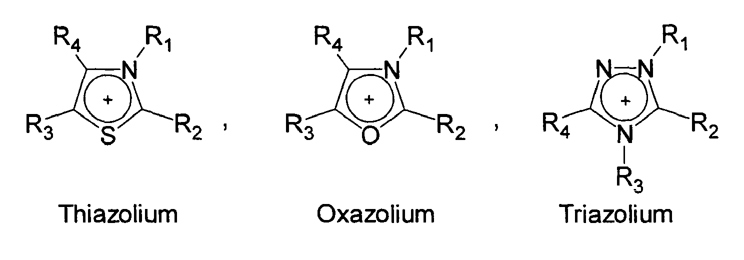 ausgewählt ist, worin R₁, R₂, R₃, R₄, R₅ und R₆ entweder H, F, jeweils getrennte Alkylgruppen mit 1 bis 4 Kohlenstoffatomen oder miteinander verbunden sind, um einen einheitlichen Alkylenrest mit 2 bis 4 Kohlenstoffatomen unter Bildung einer Ringstruktur, die am N zusammenläuft, zu bilden, oder getrennte Phenylgruppen sind, und wobei die Alkylgruppen, Alkylenreste oder Phenylgruppen mit elektronenziehenden Gruppen substituiert sein können, und
wobei das Anion ein Nicht-Lewissäure-enthaltendes polyatomares Anion mit einem van-der-Waals-Volumen ist, das 100 A³ übersteigt; vorausgesetzt, dass Lösungen von Verbindungen die durch die Formel (1/nM)⁺(ZY)₂N]⁻, (1/nM)⁺(ZY)₃C]⁻, (1/nM)⁺(ZY)₂CQ]⁻ dargestellt werden, worin M ein Kation, ausgewählt aus Pyridinen und Imidazolen, darstellt, Y SO₂ oder POZ darstellt, Q H, COZ oder Z darstellt, und jeder Substituent Z unabhängig ein Fluoratom oder eine organische Einheit darstellt, die perfluoriert sein kann oder nicht, wobei die Einheit optional zumindest eine polymerisierbare Funktion enthält, vorausgesetzt, dass zumindest einer der Z-Substituenten ein Fluoratom darstellt, in einem aprotischen Lösungsmittel, und Methidsalze ausgenommen sind.

18. Hydrophobe ionische Flüssigkeit gemäss Ansprüchen 1 bis 10, umfassend ein Kation und ein Anion, wobei das Kation ein perfluorierter, ungesättigter Stickstoff enthaltender Heterocyclus ist und das Anion organisch oder anorganisch ist.

## Revendications

1. Liquide ionique hydrophobe comprenant un cation et un anion, dans lequel ledit cation est choisi dans le groupe constitué de : 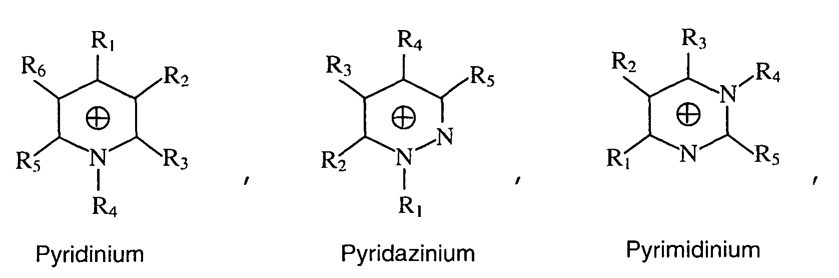 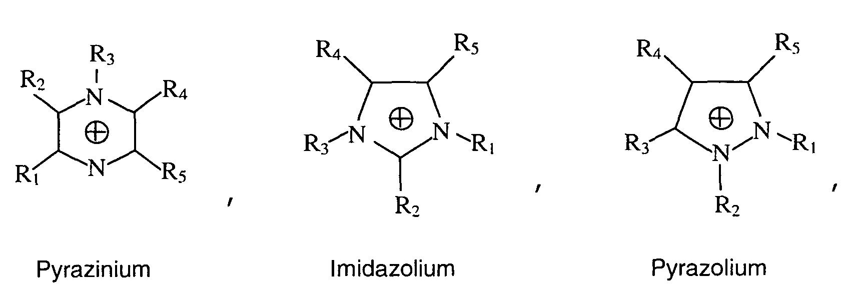 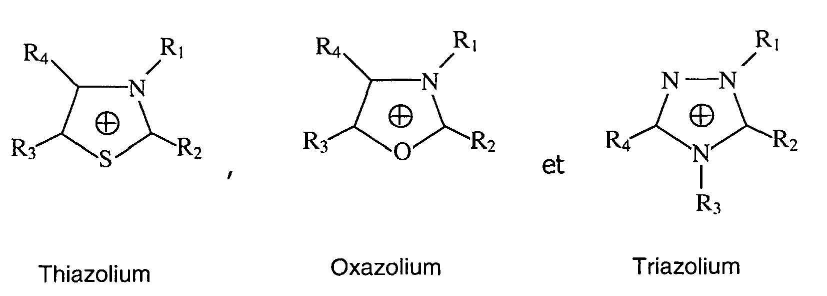 dans lesquels R₁, R₂, R₃, R₄, R₅ et R₆ représentent soit H ; soit F ; soit des groupes alkyles séparés ayant entre 1 et 4 atomes de carbone, respectivement, ou reliés entre eux pour constituer un radical alkylène unitaire ayant 2 à 4 atomes de carbone formant une structure cyclique convergeant sur N ; soit des groupes phényles séparés ; et dans lesquels les groupes alkyles, les radicaux alkylènes ou les groupes phényles peuvent être substitués par des groupes attracteurs d'électrons ; et
dans lequel ledit anion est un anion polyatomique contenant un acide qui n'est pas un acide de Lewis et ayant un volume de Van der Waals dépassant 100 Å³, pour autant que
a. les composés de formule générale : 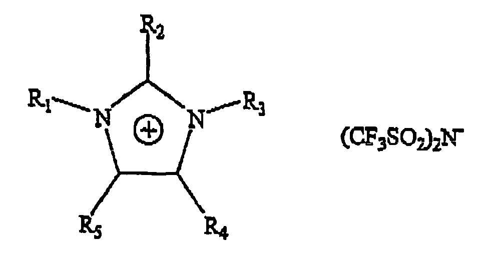 dans laquelle R₁ et R₃ sont identiques ou différents et chacun représente un radical alkyle linéaire ou ramifié, un radical fluoroalkyle ou un radical alcoxyalkyle de 1 à 4 carbones et R₂, R₄ et R₅ sont identiques ou différents et chacun représente un atome d'hydrogène ou un radical alkyle ayant de 1 à 3 atomes de carbone,
b. les composés perfluorobutylsulfonate de M-méthylpyridinium, perfluorooctanesulfonate de M-méthylpyridinium et le perfluorobutylsulfonate de N,N-diméthylimidazolium et
c. les sels méthylure.
sont exclus

2. Liquide ionique hydrophobe de la revendication 1 dans lequel, dans ledit cation, un groupe substituant attracteur d'électrons est choisi dans le groupe constitué de F-, Cl-, CF₃-, SF₅-, CF₃S-, (CF₃)₂CHS- et (CF₃)₃CS-.

3. Liquide ionique hydrophobe de la revendication 1 dans lequel ledit cation a la formule 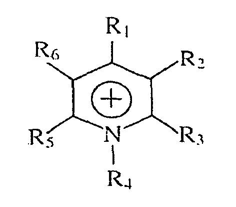 dans laquelle R₁, R₂, R₃, R₄, R₅ et R₆ représentent soit H ; soit F ; soit des groupes alkyles séparés ayant entre 1 et 4 atomes de carbone, respectivement, ou reliés entre eux pour constituer un radical alkylène unitaire ayant 2 à 4 atomes de carbone formant une structure cyclique convergeant sur N ; soit des groupes phényles séparés ; et dans laquelle les groupes alkyles, les radicaux alkylènes ou les groupes phényles peuvent être substitués par des groupes attracteurs d'électrons.

4. Liquide ionique hydrophobe de la revendication 1 dans lequel ledit cation a la formule 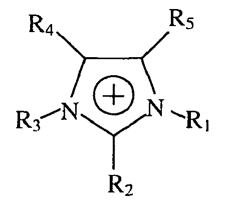 dans laquelle R₁, R₂, R₃, R₄ et R₅ représentent soit H ; soit F ; soit des groupes alkyles séparés ayant entre 1 et 4 atomes de carbone, respectivement, ou reliés entre eux pour constituer un radical alkylène unitaire ayant 2 à 4 atomes de carbone formant une structure cyclique convergeant sur N ; soit des groupes phényles séparés ; et dans laquelle les groupes alkyles, les radicaux alkylènes ou les groupes phényles peuvent être substitués par des groupes attracteurs d'électrons.

5. Liquide ionique hydrophobe de la revendication 1 dans lequel ledit cation a la formule 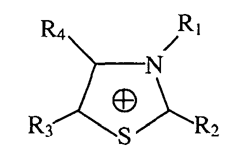 dans laquelle R₁, R₂, R₃, R₄ et R₅ représentent soit H ; soit F ; soit des groupes alkyles séparés ayant entre 1 et 4 atomes de carbone, respectivement, ou reliés entre eux pour constituer un radical alkylène unitaire ayant 2 à 4 atomes de carbone formant une structure cyclique convergeant sur N ; soit des groupes phényles séparés ; et dans laquelle les groupes alkyles, les radicaux alkylènes ou les groupes phényles peuvent être substitués par des groupes attracteurs d'électrons.

6. Liquide ionique hydrophobe de la revendication 1 dans lequel ledit cation a la formule 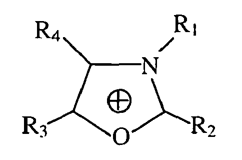 dans laquelle R₁, R₂, R₃, R₄ et R₅ représentent soit H ; soit F ; soit des groupes alkyles séparés ayant entre 1 et 4 atomes de carbone, respectivement, ou reliés entre eux pour constituer un radical alkylène unitaire ayant 2 à 4 atomes de carbone formant une structure cyclique convergeant sur N ; soit des groupes phényles séparés ; et dans laquelle les groupes alkyles, les radicaux alkylènes ou les groupes phényles peuvent être substitués par des groupes attracteurs d'électrons.

7. Liquide ionique hydrophobe de la revendication 1 dans lequel ledit anion a la formule 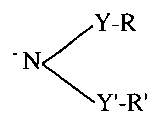 dans laquelle chacune des entités Y et Y' sont -SO₂- ou - CO- ; et les groupes R et R' sont des groupes alkyles halogénés séparés ayant 1 à 4 atomes de carbone.

8. Liquide ionique hydrophobe de la revendication 1 dans lequel ledit anion est le perfluoro-1,1-diméthylpropylate ayant la formule CF₃CF₂(CF3)₂CO'.

9. Liquide ionique hydrophobe de la revendication 1 dans lequel ledit anion est choisi dans le groupe constitué de (CF₃)₂PF₄⁻ ; (CF₃)₃PF₃⁻ ; (CF₃)₄PF₂⁻ ; (CF₃)₅PF⁻ ; (CF₃)₆P⁻ ; SF₅CF₂SO₃⁻ ; SF₅CHFCF₂SO₃⁻ ; et [O(CF₃)₂C₂(CF₃)₂O]₂PO⁻.

10. Liquide ionique hydrophobe de la revendication 1 dans lequel ledit anion est un mono- ou un di-perfluorosulfonate.

11. Electrolyte pour utilisation dans une cellule électrochimique comprenant un liquide ionique hydrophobe comprenant un cation et un anion, dans lequel ledit cation est choisi dans le groupe constitué de : 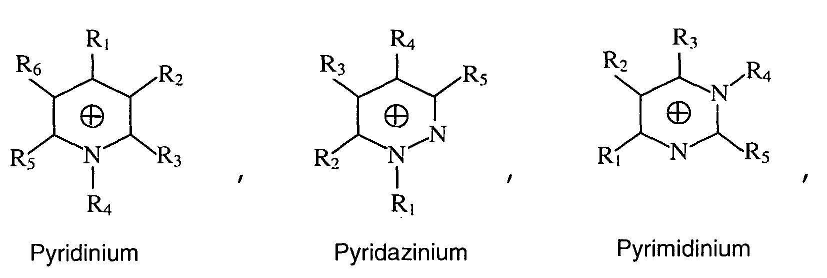 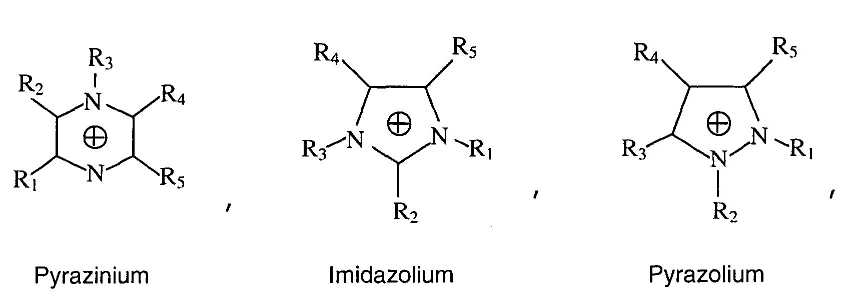 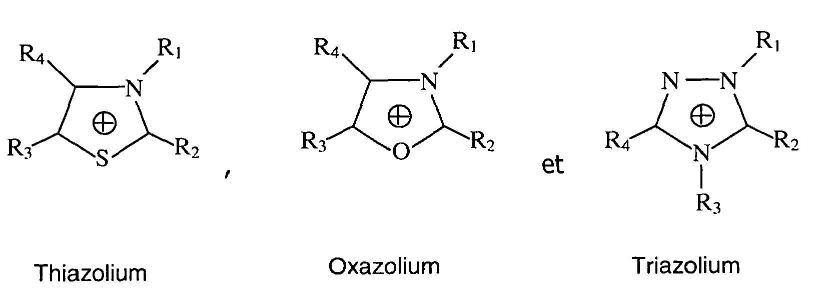 dans lesquels R₁, R₂, R₃, R₄, R₅ et R₆ représentent soit H ; soit F ; soit des groupes alkyles séparés ayant entre 1 et 4 atomes de carbone, respectivement, ou reliés entre eux pour constituer un radical alkylène unitaire ayant 2 à 4 atomes de carbone formant une structure cyclique convergeant sur N ; soit des groupes phényles séparés ; et dans lesquels les groupes alkyles, les radicaux alkylènes ou les groupes phényles peuvent être substitués par des groupes attracteurs d'électrons ; et
dans lequel ledit anion est un anion polyatomique contenant un acide qui n'est pas un acide de Lewis et ayant un volume de Van der Waals dépassant 100 Å³
et un liquide organique polaire, pour autant que
a. les solutions de composés de formule générale 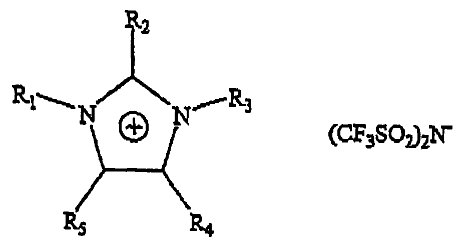 dans laquelle R₁ et R₃ sont identiques ou différents et chacun représente un radical alkyle linéaire ou ramifié, un radical fluoroalkyle ou un radical alcoxyalkyle de 1 à 4 carbones et R₂, R₄ et R₅ sont identiques ou différents et chacun représente un atome d'hydrogène ou un radical alkyle ayant de 1 à 3 atomes de carbone, dans un solvant aprotique,
b. les solutions de composés représentés par la formule (1/nM)⁺[(ZY)₂N]⁻, (1/nM)⁺[(ZY)₃C]⁻, (1/nM)⁺[(ZY)₂CQ)⁻, dans laquelle M représente un cation sélectionné parmi les pyridines et les imidazoles, Y représente SO₂ ou POZ, Q représente H, COZ ou Z et chaque substituant Z représente indépendamment un atome de fluor ou une entité organique qui peut être ou ne pas être perfluorée, laquelle entité contient facultativement au moins une fonction polymérisable, pour autant qu'au moins un des substituants Z représente un atome de fluor, dans un solvant aprotique, et
c. les sels méthylure
sont exclus.

12. Electrolyte de la revendication 11, dans lequel ledit liquide organique polaire est choisi dans le groupe constitué des éthers linéaires, des éthers cycliques, des esters, des carbonates, des lactones, des nitriles, des amides, des sulfones et des sulfolanes.

13. Electrolyte de la revendication 11, dans lequel ledit liquide organique polaire est choisi dans le groupe constitué de l'éther de diéthyle, du diméthoxyéthane, du tétrahydrofurane, du dioxane, du dioxolane, du méthyltétrahydrofurane, du formiate de méthyle, du formiate d'éthyle, du propionate de méthyle, du carbonate de propylène, du carbonate d'éthylène, du carbonate de diméthyle, du carbonate de diéthyle, du carbonate d'éthyle et de méthyle, du carbonate de dibutyle, de butyrolactones, de l'acétonitrile, du benzonitrile, du nitrométhane, du nitrobenzène, du diméthylformamide, de la N-méthylpyrrolidone, de la diméthylsulfone, de la tétraméthylènesulfone, du sulfolane et du thiophène.

14. Cellule électrochimique comprenant
une anode ;
une cathode ; et
un électrolyte comprenant un liquide ionique hydrophobe, comprenant un cation et un anion, dans lequel ledit cation est choisi dans le groupe constitué de : 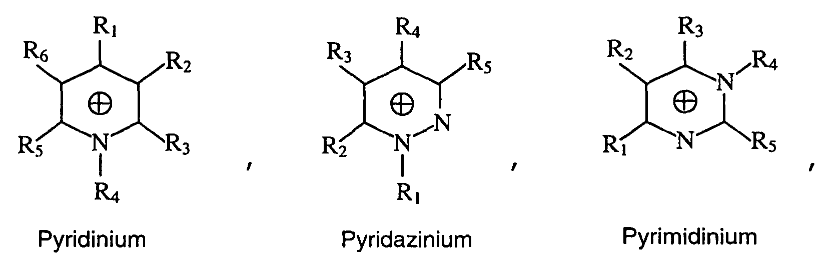 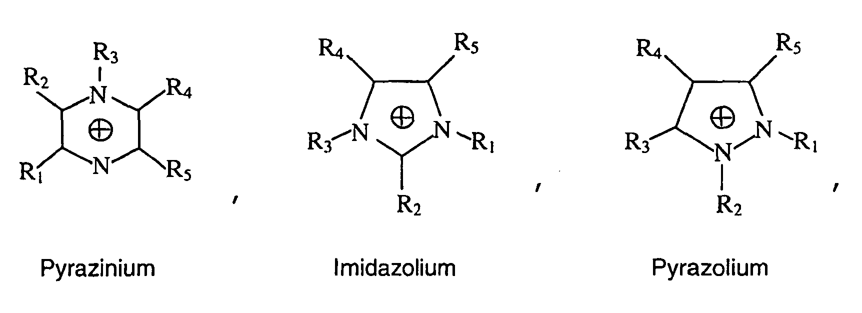 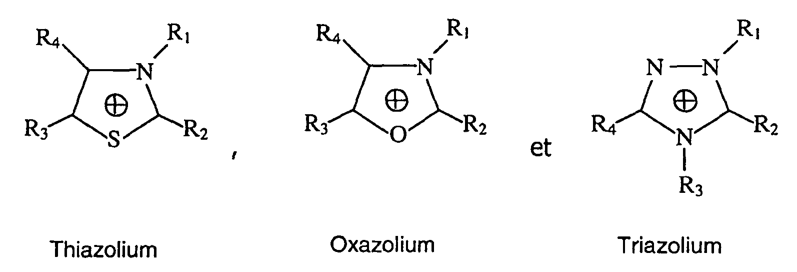 dans lesquels R₁, R₂, R₃, R₄, R₅ et R₆ représentent soit H ; soit F ; soit des groupes alkyles séparés ayant entre 1 et 4 atomes de carbone, respectivement, ou reliés entre eux pour constituer un radical alkylène unitaire ayant 2 à 4 atomes de carbone formant une structure cyclique convergeant sur N ; soit des groupes phényles séparés ; et dans lesquels les groupes alkyles, les radicaux alkylènes ou les groupes phényles peuvent être substitués par des groupes attracteurs d'électrons ; et
dans lequel ledit anion est un anion polyatomique contenant un acide qui n'est pas un acide de Lewis et ayant un volume de Van der Waals dépassant 100 Å³,
pour autant que
a. les composés de formule générale 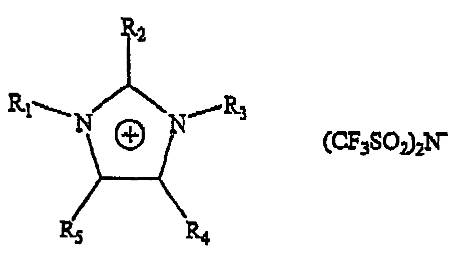 dans laquelle R₁ et R₃ sont identiques ou différents et chacun représente un radical alkyle linéaire ou ramifié, un radical fluoroalkyle ou un radical alcoxyalkyle de 1 à 4 carbones et R₂, R₄ et R₅ sont identiques ou différents et chacun représente un atome d'hydrogène ou un radical alkyle ayant de 1 à 3 atomes de carbone, dans un solvant aprotique,
b. les solutions de composés représentés par la formule (1/nM)⁺[(ZY)₂N]⁻, (1/nM)⁺[(ZY)₃C]⁻, (1/nM)⁺[(ZY)₂CQ)⁻, dans laquelle M représente un cation sélectionné parmi les pyridines et les imidazoles, Y représente SO₂ ou POZ, Q représente H, COZ ou Z et chaque substituant Z représente indépendamment un atome de fluor ou une entité organique qui peut être ou ne pas être perfluorée, laquelle entité contient facultativement au moins une fonction polymérisable, pour autant qu'au moins un des substituants Z représente un atome de fluor, dans un solvant aprotique, et
c. les sels méthylure
sont exclus.

15. Cellule électrochimique comprenant
une anode ;
une cathode ; et
un électrolyte comprenant un liquide ionique hydrophobe comprenant un cation et un anion, dans lequel ledit cation est choisi dans le groupe constitué de : 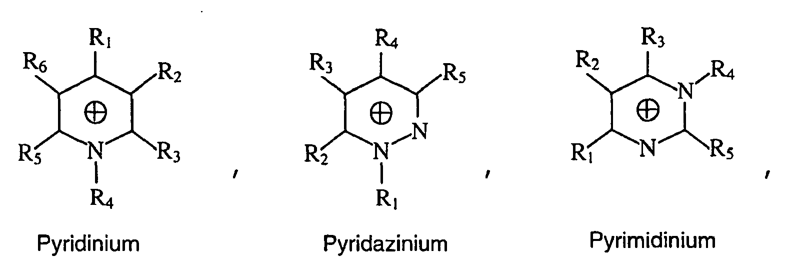 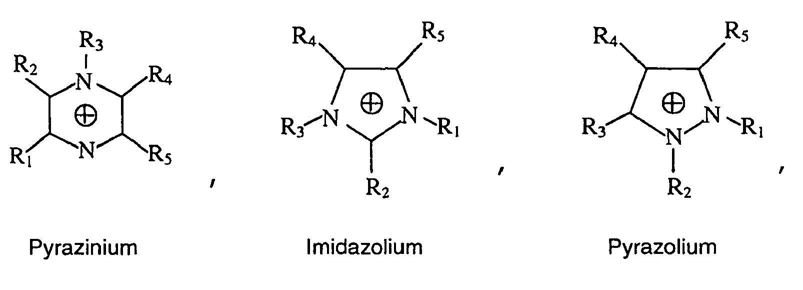 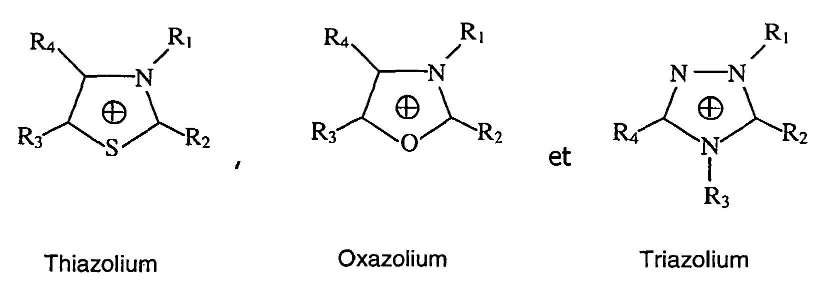 dans lesquels R₁, R₂, R₃, R₄, R₅ et R₆ représentent soit H ; soit F ; soit des groupes alkyles séparés ayant entre 1 et 4 atomes de carbone, respectivement, ou reliés entre eux pour constituer un radical alkylène unitaire ayant 2 à 4 atomes de carbone formant une structure cyclique convergeant sur N ; soit des groupes phényles séparés ; et dans lesquels les groupes alkyles, les radicaux alkylènes ou les groupes phényles peuvent être substitués par des groupes attracteurs d'électrons ; et
dans lequel ledit anion est un anion polyatomique contenant un acide qui n'est pas un acide de Lewis et ayant un volume de Van der Waals dépassant 100 Å³.
et un sel de lithium.

16. Cellule électrochimique de la revendication 14 ou de la revendication 15, dans laquelle ledit électrolyte comprend en plus un liquide organique polaire.

17. Condensateur comprenant
une première électrode ;
une deuxième électrode ; et
un électrolyte, ledit électrolyte comprenant un liquide ionique hydrophobe comprenant un cation et un anion, dans lequel ledit cation est choisi dans le groupe constitué de : 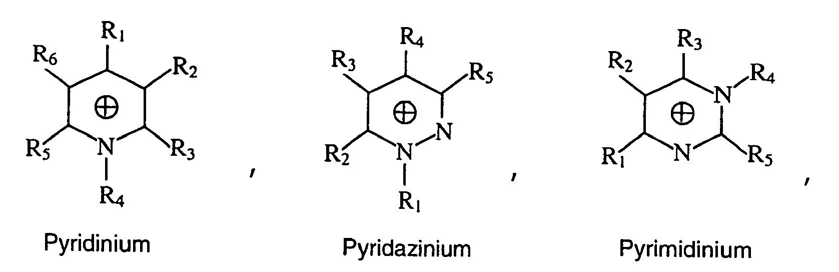 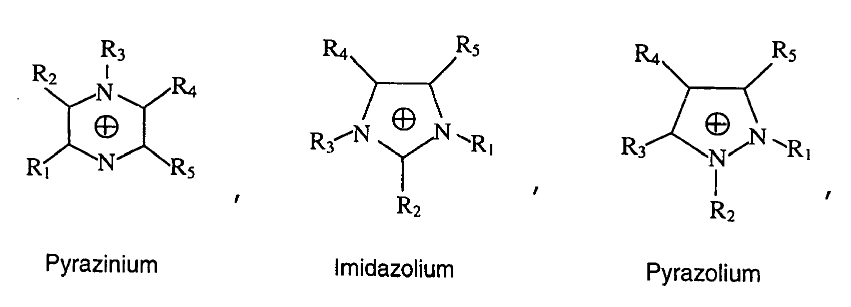 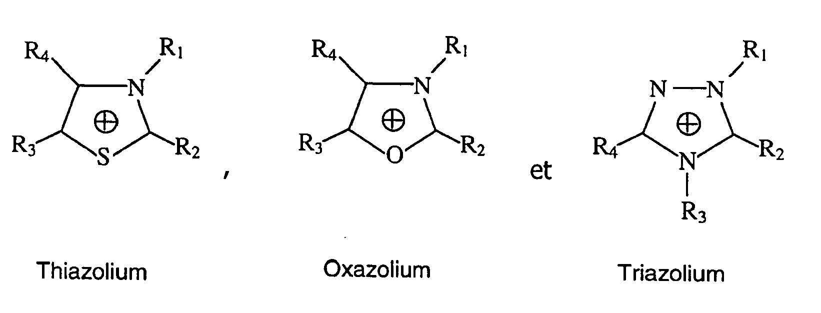 dans lesquels R₁, R₂, R₃, R₄, R₅ et R₆ représentent soit H ; soit F ; soit des groupes alkyles séparés ayant entre 1 et 4 atomes de carbone, respectivement, ou reliés entre eux pour constituer un radical alkylène unitaire ayant 2 à 4 atomes de carbone formant une structure cyclique convergeant sur N ; soit des groupes phényles séparés ; et dans lesquels les groupes alkyles, les radicaux alkylènes ou les groupes phényles peuvent être substitués par des groupes attracteurs d'électrons ; et
dans lequel ledit anion est un anion polyatomique contenant un acide qui n'est pas un acide de Lewis et ayant un volume de Van der Waals dépassant 100 Å³,
pour autant que
les solutions de composés représentés par la formule (1/nM)⁺[(ZY)₂N]⁻, (1/nM)⁺[(ZY)₃C]⁻, (1/nM)⁺[(ZY)₂CQ)⁻, dans laquelle M représente un cation sélectionné parmi les pyridines et les imidazoles, Y représente SO₂ ou POZ, Q représente H, COZ ou Z et chaque substituant Z représente indépendamment un atome de fluor ou une entité organique qui peut être ou ne pas être perfluorée, laquelle entité contient facultativement au moins une fonction polymérisable, pour autant qu'au moins un des substituants Z représente un atome de fluor, dans un solvant aprotique, et les sels méthylure sont exclus.

18. Liquide ionique hydrophobe selon les revendications 1-10, comprenant un cation et un anion, dans lequel ledit cation est un hétérocycle contenant de l'azote, insaturé et perfluoré et ledit anion est organique ou inorganique.
